(19) Europäisches Patentamt
European Patent Office
Office européen des brevets

(11) Veröffentlichungsnummer: **0 059 158**
**B1**

(12) # EUROPÄISCHE PATENTSCHRIFT

(45) Veröffentlichungstag der Patentschrift: **03.08.88**

(21) Anmeldenummer: **82730015.3**

(22) Anmeldetag: **19.02.82**

(51) Int. Cl.⁴: **C 07 D 209/52**, C 07 D 307/935, C 07 C 177/00, A 61 K 31/40, A 61 K 31/557 // C07D407/12, C07C69/52, C07C57/00, C07F9/40

(54) **Azaprostacycline, Verfahren zu ihrer Herstellung und ihre pharmazeutische Verwendung.**

(30) Priorität: **20.02.81 DE 3107100**

(43) Veröffentlichungstag der Anmeldung: **01.09.82 Patentblatt 82/35**

(45) Bekanntmachung des Hinweises auf die Patenterteilung: **03.08.88 Patentblatt 88/31**

(84) Benannte Vertragsstaaten: **AT BE CH DE FR GB IT LI LU NL SE**

(56) Entgegenhaltungen: **keine**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Schwarz, Norbert, Dr., Hauptstrasse 118, D-1000 Berlin 62 (DE)**
Erfinder: **Skuballa, Werner, Dr., Olwenstrasse 25, D-1000 Berlin 28 (DE)**
Erfinder: **Vorbrüggen, Helmut, Prof. Dr., Wilkestrasse 7, D-1000 Berlin 27 (DE)**
Erfinder: **Casals-Stenzel, Jorge, Dr., Wichernstrasse 20, D-1000 Berlin 33 (DE)**
Erfinder: **Schillinger, Ekkehard, Dr., Im Amseltal 50, D-1000 Berlin 28 (DE)**
Erfinder: **Town, Michael Harold, Dr., Buchsbaumweg 3, D-1000 Berlin 47 (DE)**

EP 0 059 158 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft neue Prostacyclin-Derivate, Verfahren zu ihrer Herstellung sowie ihre Verwendung als Arzneimittel.

Prostacyclin ($PGI_2$), einer der Hauptfaktoren bei der Blutplättchenaggregation, wirkt dilatierend auf verschiedene Blutgefässe (Science 196, 1072) und ist daher als Mittel zur Blutdrucksenkung anzusehen. $PGI_2$ besitzt jedoch nicht die für ein Arzneimittel notwendige Stabilität. So beträgt die Halbwertzeit von $PGI_2$ bei physiologischen pH-Werten und bei Raumtemperatur nur wenige Minuten.

In einer Publikation der Firma Upjohn (G.L. Bundy et al.), Tetrahedron Letters 1978, 1371) sowie in der DE-A-2 826 096 wird über 9α,6-Nitrilo-prostaglandine berichtet.

In den Schutzrechten EP-A-15 227, DE-A-2 734 791 und GB-A-2 033 386 werden Aza- bzw. Oxacarbacycline, die sich in der Stellung der ungesättigten Gruppen in der unteren Kette von den neuen Azaprostacyclinen unterscheiden sowie entsprechende Zwischenprodukte beschrieben.

Es wurde nun gefunden, dass durch Einführung von Doppelbindungen und gegebenenfalls Alkylgruppen in 19-Stellung in der unteren Kette des 9-Desoxy-9α,6-nitrilo-PGF eine längere Wirkungsdauer, grössere Selektivität und bessere Wirksamkeit erzielt werden kann.

Die erfindungsgemässen Verbindungen wirken blutdrucksenkend und bronchodilatorisch. Sie sind ausserdem zur Inhibierung der Thrombozytenaggregation geeignet.

Die Erfindung betrifft Azaprostacycline der allgemeinen Formel I

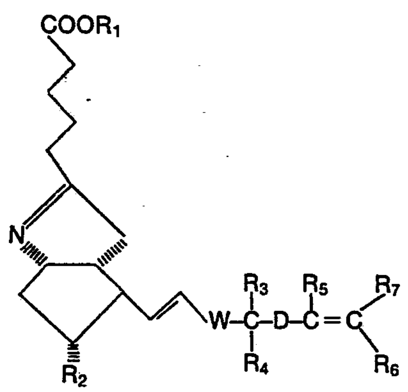

(I),

worin

$R_1$ Wasserstoff oder Alkyl mit 1–5 C-Atomen,

W eine CHOH-Gruppe, wobei die OH-Gruppe α- oder β-ständig sein kann,

$R_2$ eine Hydroxygruppe,

$R_3$ und $R_4$ Wasserstoff oder eine Alkylgruppe mit 1–5 C-Atomen,

D eine Alkylengruppe mit 1–2 C-Atomen,

$R_5$ Wasserstoff oder Alkyl mit 1–2 C-Atomen oder, wenn D eine Alkylengruppe mit 2 C-Atomen darstellt, zusammen mit $R_6$ eine Bindung,

$R_6$ und $R_7$ Wasserstoff oder Alkyl mit 1–2 C-Atomen,

$R_6(R_7)$ Halogen, wenn $R_7(R_6)$ Alkyl mit 1–2 C-Atomen darstellt oder

$R_5$ und $R_7$ Wasserstoff oder Alkyl mit 1–2 C-Atomen und

D und $R_6$ Glieder eines über $(CH_2)_{1-3}$ geschlossenen Ringes mit D als $-CH<$ und $R_6$ als $-CH_2-$ sind und,

falls $R_1$ die Bedeutung eines Wasserstoffatoms hat, deren Salze mit physiologisch verträglichen Basen.

Als Alkylgruppe $R_1$ sind gerade oder verzweigte Alkylgruppen mit 1–5 C-Atomen zu betrachten, wie beispielsweise Methyl, Äthyl, Propyl, Butyl, Isobutyl, tert.-Butyl, Pentyl oder Neopentyl.

Als bevorzugte Alkylgruppen $P_1$ sind solche mit 1–4 C-Atomen, wie z.B. Methyl, Äthyl, Propyl, Dimethylaminopropyl, Isobutyl, Butyl zu nennen.

Als Alkylgruppen $R_3$ und $R_4$ kommen gerad- und verzweigtkettige Alkylreste mit 1–5 Kohlenstoffatomen in Betracht, wie beispielsweise der Methyl-, Äthyl-, Propyl-, Isopropyl-, Butyl-, Isobutyl-, tert.-Butyl-, Pentyl-, Isopentyl- und Neopentyl-Rest. Bevorzugt sind die Methyl- und die Äthylgruppe.

Als Halogene $R_6$ oder $R_7$ kommen Fluor, Chlor, Brom und Jod in Betracht. Bevorzugt sind Fluor und Chlor.

Zur Salzbildung mit den freien Säuren ($R_1$ = H) sind anorganische und organische Basen geeignet, wie sie dem Fachmann zur Bildung physiologisch verträglicher Salze bekannt sind. Beispielsweise seien genannt: Alkalihydroxide, wie Natrium- und Kaliumhydroxid, Erdalkalihydroxide, wie Calciumhydroxid, Ammoniak, Amine, wie Äthanolamin, Diäthanolamin, Triäthanolamin, N-Methylglucamin, Morpholin, Tris-(hydroxymethyl)-methylamin, Aminosäuren, wie Arginin, usw.

Die Erfindung betrifft ferner ein Verfahren zur Herstellung der erfindungsgemässen Azaprostacycline der allgemeinen Formel I, dadurch gekennzeichnet, dass man in an sich bekannter Weise eine Verbindung der allgemeinen Formel II

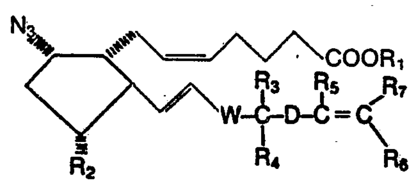

(II),

worin $R_1$, $R_2$, $R_3$, $R_4$, $R_5$, $R_6$, $R_7$, W und D die obengenannten Bedeutungen haben, in einem inerten Lösungsmittel einer thermischen Behandlung unterwirft und gegebenenfalls anschliessend in beliebiger Reihenfolge geschützte Hydroxygruppen freisetzt und/oder freie Hydroxygruppen verestert, veräthert und/oder eine freie Carboxylgruppe verestert und/oder eine veresterte Carboxylgruppe verseift oder eine Carboxylgruppe mit einer physiologisch verträglichen Base in ein Salz überführt.

Die thermische Umsetzung der Verbindung der allgemeinen Formel II wird bei Temperaturen von 20° bis 150 °C, vorzugsweise 40°–120 °C, vorgenommen. Für den bevorzugten Temperaturbereich kommen beispielsweise folgende inerte Lösungsmittel in Frage: Essigsäureäthylester, Essigsäuremethylester, Tetrahydrofuran, Dimethoxyäthan, Tetrachlorkohlenstoff, Methylenchlorid, 1,2-Dichloräthan, Dimethylformamid usw.

Die Verseifung der Prostaglandinester wird nach den dem Fachmann bekannten Methoden durchgeführt, beispielsweise mit basischen Katalysatoren. Die Einführung der Estergruppe, bei der $R_1$ eine Alkylgruppe mit 1–5 C-Atomen darstellt, erfolgt nach den dem Fachmann bekannten Methoden. Die Carboxyverbindungen werden beispielsweise mit Diazokohlenwasserstoffen in an sich bekannter Weise umgesetzt. Die Veresterung mit Diazokohlenwasserstoffen erfolgt zum Beispiel dadurch, dass man eine Lösung des Diazokohlenwasserstoffs in einem inerten Lösungsmittel, vorzugsweise in Diäthyläther, mit der Carboxyverbindung in dem gleichen oder in einem anderen inerten Lösungsmittel, wie z.B. Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org. Reactions Bd. 8, Seiten 389–394 (1954)].

Die Prostacyclin-Derivate der allgemeinen Formel I mit $R_1$ in der Bedeutung eines Wasserstoffatoms können mit geeigneten Mengen der entsprechenden anorganischen Basen unter Neutralisierung in Salze überführt werden. Beispielsweise erhält man beim Lösen der entsprechenden PG-Säuren in Wasser, welches die stöchiometrische Menge der Base enthält, nach Abdampfen des Wassers oder nach Zugabe eines mit Wasser mischbaren Lösungsmittels, zum Beispiel Alkohol oder Aceton, das feste anorganische Salz.

Zur Herstellung eines Aminsalzes, die in üblicher Weise erfolgt, wird die PG-Säure zum Beispiel in einem geeigneten Lösungsmittel, beispielsweise Äthanol, Aceton, Diäthyläther oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die funktionelle Abwandlung der freien OH-Gruppen erfolgt nach den dem Fachmann bekannten Methoden. Zur Einführung der Ätherschutzgruppen wird beispielsweise mit Dihydropyran in Methylenchlorid oder Chloroform unter Verwendung eines sauren Kondensationsmittels, wie zum Beispiel p-Toluolsulfonsäure, umgesetzt. Das Dihydropyran wird im Überschuss angewandt, vorzugsweise in der 2- bis 10fachen Menge des theoretischen Bedarfs. Die Umsetzung ist normalerweise bei 0°–30 °C nach 15–30 Minuten beendet. Die Einführung der Acylschutzgruppen erfolgt, indem man eine Verbindung der allgemeinen Formel I in an sich bekannter Weise mit einem Carbonsäurederivat, wie zum Beispiel Säurechlorid, Säureanhydrid u.a., umsetzt.

Die Freisetzung einer funktionell abgewandelten OH-Gruppe zu den Verbindungen der allgemeinen Formel I erfolgt nach bekannten Methoden. Beispielsweise wird die Abspaltung von Ätherschutzgruppen in einer wässrigen Lösung einer organischen Säure, wie zum Beispiel Essigsäure, Propionsäure u.a., durchgeführt. Zur Verbesserung der Löslichkeit wird zweckmässigerweise ein mit Wasser mischbares inertes organisches Lösungsmittel zugesetzt. Geeignete organische Lösungsmittel sind zum Beispiel Alkohole, wie Methanol und Äthanol, und Äther, wie Dimethoxyäthan, Dioxan und Tetrahydrofuran. Tetrahydrofuran wird bevorzugt angewendet. Die Abspaltung wird vorzugsweise bei Temperaturen zwischen 20° und 80 °C durchgeführt.

Die Abspaltung der Silylätherschutzgruppen erfolgt beispielsweise mit Tetrabutylammoniumfluorid. Als Lösungsmittel sind beispielsweise geeignet Tetrahydrofuran, Diäthyläther, Dioxan, Methylenchlorid usw. Die Abspaltung wird vorzugsweise bei Temperaturen zwischen 0° und 80 °C durchgeführt.

Die Verseifung der Acylgruppen erfolgt beispielsweise mit Alkali- oder Erdalkalicarbonaten oder -hydroxyden in einem Alkohol oder der wässrigen Lösung eines Alkohols. Als Alkohole kommen aliphatische Alkohole in Betracht, wie zum Beispiel Methanol, Äthanol, Butanol usw., vorzugsweise Methanol. Als Alkalicarbonate und -hydroxyde seien Kalium- und Natriumsalze genannt, bevorzugt sind jedoch die Kaliumsalze. Als Erdalkalicarbonate und -hydroxyde sind beispielsweise geeignet Calciumcarbonat, Calciumhydroxyd und Bariumcarbonat. Die Umsetzung erfolgt bei −10° bis 70 °C, vorzugsweise bei 25 °C.

Das als Ausgangsmaterial für das vorstehend beschriebene Verfahren verwendete Azid der allgemeinen Formel II kann hergestellt werden, indem man einen Alkohol der allgemeinen Formel III

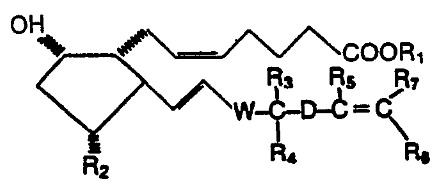

(III)

(DE-A-2 729 960), worin freie Hydroxygruppen in $R_2$ und W beispielsweise als Tetrahydropyranyläther geschützt sind, mit p-Toluolsulfonsäurechlorid in das Tosylat der allgemeinen Formel IV überführt.

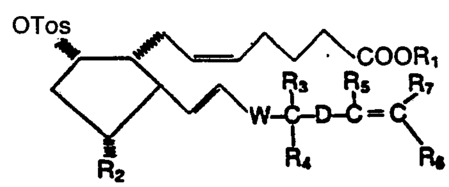

(IV)

Durch Umsetzung mit Kaliumnitrit in Dimethylsulfoxid erhält man den 9-β-konfigurierten Alkohol V, den man mit p-Toluol-

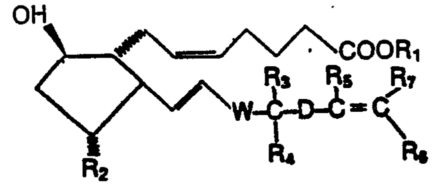

(V)

sulfonsäurechlorid in Gegenwart von Pyridin zum Tosylat der allgemeinen Formel VI umsetzt.

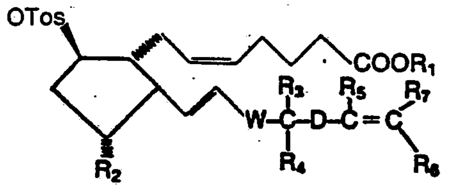

(VI)

Auf dieser Stufe kann man gegebenenfalls die Tetrahydropyranylätherschutzgruppe abspalten. Das Tosylat wird anschliessend mit Natriumazid in einem polaren, aprotischen Lösungsmittel wie DMF, N-Methylpyrrolidon, oder vorzugsweise HMPT (Hexamethylphosphorsäuretriamid) in das Azid der allgemeinen Formel II überführt, das gegebenenfalls verseift wird ($R_1$ = H).

Die Verbindungen dieser Erfindung wirken blutdrucksenkend und bronchodilatorisch. Die neuen Prostacyclin-Derivate der Formel I stellen damit wertvolle pharmazeutische Wirkstoffe dar. Darüber hinaus weisen sie bei ähnlichem Wirkungsspektrum verglichen mit entsprechenden Prostaglandinen, eine höhere Spezifität und vor allem eine wesentlich längere Wirksamkeit auf. Im Vergleich zu $PGI_2$ zeichnen sie sich durch grössere Stabilität aus. Die hohe Gewebsspezifität der neuen Prostaglandine zeigt sich bei der Untersuchung an glattmuskulären Organen, wie z.B. am Meerschweinchenileum oder an der isolierten Kaninchentrachea, wo eine wesentlich geringere Stimulation zu beobachten ist als bei der Applikation natürlicher Prostaglandine vom E-, A- oder F-Typ. Gegenüber vergleichbaren Prostacyclinen zeichnen sich einige dieser neuen Azaprostacycline durch eine starke Wirkungsdissoziation aus. Sie senken den Blutdruck bei nur geringer Inhibierung der Thrombozytenaggregation.

Die neuen Prostacyclin-Analoga besitzen die für Prostacycline typischen Eigenschaften, wie z.B. Senkung des peripheren arteriellen und koronaren vaskulären Widerstandes, myocardiale Zytoprotektion und damit Senkung des systemischen Blutdrucks, ohne zugleich Schlagvolumen und koronare Durchblutung zu senken; Behandlung von Schlaganfall, Prophylaxe und Therapie koronarer Herzerkrankungen, koronarer Thrombose, des Herzinfarkts, peripherer Arterienerkrankungen, Arteriosklerose und Thrombose, Prophylaxe und Therapie ischämischer Attacken des ZNS-Systems, Therapie des Schocks, Inhibierung der Bronchokonstriktion, Inhibierung der Magensäuresekretion und Zytoprotektion der Magen- und Darmschleimhaut, Zytoprotektion in der Leber und im Pankreas; antiallergische Eigenschaften, Senkung des pulmonaren vaskulären Widerstandes und des pulmonaren Blutdrucks, Förderung der Nierendurchblutung, Anwendung anstelle von Heparin oder als Adjuvans bei der Dialyse der Hämofiltration, Inhibierung von Geburtswehen, Behandlung von Schwangerschaftstoxikose, Erhöhung der zerebralen Durchblutung etc. Ausserdem besitzen die neuen Prostaglandinanaloga antiproliferative und antidiarrhoegene Eigenschaften. Die neuen Prostacycline können auch in Kombination verwendet werden, z.B. mit β-Blockern und Diuretika.

Die Dosis der Verbindungen ist 1–1500 µg/kg/Tag, wenn sie am menschlichen Patienten verabreicht werden. Die Einheitsdosis für den pharmazeutischen akzeptablen Träger beträgt 0,01–100 mg.

Bei intravenöser Injektion an wachen, hypertonen Ratten in Dosen von 5, 20 und 100 µg/kg Körpergewicht zeigen die erfindungsgemässen Verbindungen eine stärkere blutdrucksenkende und länger anhaltende Wirkung als $PGE_2$ und $PGA_2$, ohne wie $PGE_2$ Durchfälle oder $PGA_2$ kardiale Arrhythmien auszulösen.

Bei intravenöser Injektion an narkotisierten Kaninchen zeigen die erfindungsgemässen Verbindungen im Vergleich zu $PGE_2$ und $PGA_2$ eine stärkere und erheblich länger anhaltende Blutdrucksenkung, ohne dass andere glattmuskuläre Organe oder Organfunktionen beeinflusst werden.

Für die parenterale Verabreichung werden sterile, injizierbare, wässrige oder ölige Lösungen benutzt. Für die orale Applikation sind beispielsweise Tabletten, Dragées oder Kapseln geeignet.

Die Erfindung betrifft damit auch Arzneimittel auf Basis der Verbindungen der allgemeinen Formel I und üblicher Hilfs- und Trägerstoffe.

Die erfindungsgemässen Wirkstoffe sollen in Verbindung mit den in der Galenik bekannten und üblichen Hilfsstoffen z.B. zur Herstellung von Blutdrucksenkern dienen.

Beispiel 1

(13E)-(11R,15S,16RS)-11,15-Dihydroxy-16,19-dimethyl-9α,6-nitrilo-13,18-prostadiensäure

Eine Lösung von 298 mg
(5Z,13E)-(8R,9S,11R,12R,15S,16RS)-9-Azido-11,15-dihydroxy-16,19-dimethyl-5,13,18-prostadiensäure
in 28 ml Essigsäureäthylester wurde 27 Stunden bei 80 °C unter Argon gerührt. Anschliessend dampfte man im Vakuum ein und reinigte den Rückstand durch Säulenchromatographie an Kieselgel mit Essigester/0–50% Methanol als Fliessmittel. Es wurden 172 mg der Titelverbindung als viskoses Öl erhalten.

IR: 3380 (breit), 2960, 2930, 2870, 1710, 1640, 1085, 1020, 970/cm.

Das Ausgangsmaterial für die obige Titelverbindung wurde wie folgt hergestellt:

1a) 2-Äthoxycarbonyl-2,5-dimethyl-4-hexensäureäthylester

In einem mit Rückflusskühler, Tropftrichter und Rührer versehenen Dreihalskolben wurden 36,1 g Natrium (in kleine Stücke geschnitten) gegeben. Hierzu wurden 800 ml abs. Äthanol so schnell zugetropft, dass die Lösung in lebhaftem Sieden blieb. Zur heissen Alkoholat-Lösung tropfte man 269,6 g frisch destillierten Methylmalonsäurediäthylester, rührte 1/2 Stunde bei 60 °C und gab dann ebenfalls tropfenweise 241,7 g Dimethylallylbromid hinzu. Nach einer Stunde Rühren unter Erhitzen filtrierte man das ausgefallene Natriumbromid ab, wusch den Niederschlag und engte das Filtrat ein. Der Rückstand wurde in Äther aufgenommen, mit gesättigter Natriumchlorid-Lösung neutral gewaschen, über Magnesiumsulfat getrocknet und am Rotationsverdampfer eingeengt. Der Eindampfrückstand wurde an der Ölpumpe fraktioniert. Man erhielt 266 g der Titelverbindung vom $Kp_7$ = 97–112 °C.

IR (Film): 1735, 1245, 1025, 860/cm.

1b) 2-Carboxy-2,5-dimethyl-4-hexensäure

223,8 g des in der vorigen Reaktionsstufe erhaltenen Diesters wurden zusammen mit 181 g Kaliumhydroxid in 235 ml Wasser und 450 ml Äthanol 4 Stunden unter Rückfluss erhitzt. Anschliessend wurde das Äthanol am Rotationsverdampfer abgezogen, der Rückstand in 235 ml Wasser gelöst und unter Eiskühlung mit konz. Salzsäure bis zum pH-Wert 1 tropfenweise versetzt. Der Niederschlag (Fp 162–166 °C) wurde gesammelt, mit Wasser gewaschen und ohne weitere Reinigung in die nächste Stufe eingesetzt.

IR (KBr): 1700, 1230, 840/cm.

1c) 2,5-Dimethyl-4-hexensäure

Die in der vorigen Reaktionsstufe erhaltene Dicarbonsäure wurde in einer Destillationsapparatur 4 Stunden bei Normaldruck, anschliessend eine Stunde bei 75 Torr auf 210 °C gehalten. Das Produkt wurde dann im Vakuum destilliert. Es wurden 68 g der Titelverbindung ($Kp_5$ = 98–106 °C; $Kp_1$ = 67–70 °C) erhalten.

IR (Film): 1705, 1220, 810/cm.

1d) 2,5-Dimethyl-4-hexensäure-methylester

Die nach obiger Vorschrift erhaltenen 68 g Carbonsäure wurden mit so viel ätherischer Diazomethan-Lösung versetzt, bis sich kein Stickstoff mehr bei Zugabe des Reagenzes entwickelte und die Gelbfärbung der Reaktionslösung bestehen blieb. Das Lösungsmittel wurde dann im Vakuum entfernt und der Rückstand fraktioniert. Man erhielt 62,3 g vom $Kp_{3,5-6}$ = 32–35 °C.

IR (Film): 1735, 1160, 1050, 820/cm.

1e) 2,5-Dimethyl-4-hexensäure-äthylester

85,3 g 2-Äthoxycarbonyl-2,5-dimethyl-4-hexensäureäthylester wurden in 645 ml Dimethylsulfoxid gelöst und nacheinander mit 29,7 g Lithiumchlorid und 6,3 ml dest. Wasser versetzt. Danach wurde das Reaktionsgemisch insgesamt 13 Stunden auf 200 °C erhitzt und anschliessend – nach Abkühlung – auf 1 l Eiswasser gegossen. Die wässrige Phase wurde dreimal mit je 500 ml Methylenchlorid extrahiert. Die vereinigten organischen Extrakte wurden dann zweimal mit Wasser gewaschen, über Magnesiumsulfat getrocknet, am Rotationsverdampfer eingeengt und im Vakuum destilliert. Man isolierte 53,1 g vom $Kp_{13}$ = 75–78 °C.

IR (Film): 1735, 1160, 1050/cm.

1f) 2-(1,4-Dimethyl-3-pentenyl)-2-oxoäthanphosphonsäuredimethylester

Zu einer Lösung von 59 g Methanphosphonsäuredimethylester in 400 ml abs. Tetrahydrofuran tropfte man unter Argon bei −60 °C 274,7 ml einer 1,61 m Butyllithium-Lösung in Hexan. Nach 15 Minuten Nachrühren wurde eine Lösung von 34,05 g 2,5-Dimethyl-4-hexensäure-äthylester in 100 ml abs. Tetrahydrofuran zugetropft. Man liess das Reaktionsgemisch innerhalb von vier Stunden sich auf Raumtemperatur erwärmen und rührte es anschliessend noch 3 Stunden. Danach wurde es mit 26,5 ml Eisessig versetzt und im Vakuum eingeengt. Der Rückstand wurde mit Äther/Wasser aufgenommen, die wässrige Phase mit festem Natriumchlorid versetzt und ausgeäthert. Die vereinigten organischen Phasen wurden über Magnesiumsulfat getrocknet und am Rotationsverdampfer eingeengt. Der Eindampfrückstand wurde durch Säulenchromatographie an Kieselgel mit Hexan/50–100% Essigester als Fliessmittel gereinigt. Man erhielt 32 g der gewünschten Verbindung.

IR (Film): 1710, 1260, 1030/cm.

1g) (1S,5R,6R,7R)-7-Benzoyloxy-6[(E)-(4RS)-4,7-dimethyl-3-oxo-1,6-octadienyl]-2-oxabicyclo[3.3.0]-octan-3-on

Zu einer Suspension von 1,62 g 50%igem (in Öl suspendiertem) Natriumhydrid in 150 ml frisch über Lithiumaluminiumhydrid destilliertem Dimethoxyäthan wurden unter Argon bei Raumtemperatur 8,4 g des in der vorigen Reaktionsstufe erhaltenen Phosphonats (gelöst in 100 ml abs. Dimethoxyäthan) getropft. Nach Zugabe von 1,44 g Lithiumchlorid (vorher im Vakuum 2 Stunden bei 50 °C getrocknet) wurde das Reaktionsgemisch 2 Stunden bei Raumtemperatur gerührt. Anschliessend wurde die Suspension auf −20 °C abgekühlt und tropfenweise mit einer Lösung von 9,28 g (1S,5R,6R,7R)-6-Formyl-7-benzoyloxy-2-oxabicyclo[3.3.0]octan-3-on [E.J. Corey et al., J. Amer. Chem. Soc. 91, 5675 (1969)] in 250 ml abs. Dimethoxyäthan innerhalb einer halben Stunde versetzt. Danach liess man die Temperatur innerhalb von 2 Stunden unter Rühren auf 0 °C ansteigen. Nach erfolgter analytischer Dünnschichtchromatographie-Kontrolle wurden anschliessend bei −10 °C 3,4 ml Eisessig tropfenweise zugegeben. Danach versetzte man mit 450 ml Wasser, trennte die Phasen, schüttelte die wässrige Phase dreimal mit je ca. 200 ml Äther aus, vereinigte die organischen Phasen und wusch sie mit 4%iger Natriumbicarbonat- und gesättigter Natriumchloridlösung. Nach Trocknen über Magnesiumsulfat wurde das Lösungsmittel am Rotationsverdampfer entfernt. Nach Reinigung des Rückstands durch Säulenchromatographie an Kieselgel mit Hexan/20–60% Essigester als Fliessmittel erhielt man 12,1 g der Titelverbindung als farbloses Öl.

IR (Film): 1775, 1720, 1690, 1670, 1620, 1600, 1580, 1270, 1180, 710/cm.

1h) (1S,5R,6R,7R)-7-Benzoyloxy-6-[(E)-(3S,4RS)-3-hydroxy-4,7-dimethyl-1,6-octadienyl]-2-oxabicyclo-[3.3.0]-octan-3-on

Zu einer auf −40 °C gekühlten Lösung von 8,0 g des in der vorigen Reaktionsstufe erhaltenen Ketons (Beispiel 1g) in 250 ml abs. Methanol wurden portionsweise 4,8 g Natriumborhydrid gegeben. Nach 30 Minuten Nachrühren bei dieser Temperatur wurden – ebenfalls bei −40 °C – 10,37 ml Eisessig zur Reaktionslösung getropft. Nach Entfernen des Lösungsmittels am Rotationsverdampfer wurde der Rückstand mit einem Zweiphasengemisch aus 200 ml Wasser und 300 ml Methylenchlorid versetzt, die abgetrennte Wasserphase mit

festem Natriumchlorid versetzt und zweimal mit je ca. 200 ml Methylenchlorid extrahiert. Die vereinigten organischen Phasen wurden mit gesättigter Natriumchloridlösung gewaschen, über Magnesiumsulfat getrocknet und im Vakuum eingeengt. Die Isomerentrennung des Rückstands erfolgte durch Säulenchromatographie an Kieselgel mit Hexan/10–60% Essigester als Fliessmittel. Als unpolarstes Produkt wurden 4,5 g der Titelverbindung isoliert.

IR (Film): 3480 (breit), 1775, 1720, 1605, 1590, 1280, 1180, 715/cm.

1i) (1S,5R,6R,7R)-6-[(E)-(3S)-(4RS)-3-Hydroxy-4,7-dimethyl-1,6-ocadienyl]-7-hydroxy-2-oxabicyclo-[3.3.0]-octan-3-on

Eine Lösung von 10 g des in der vorigen Reaktionsstufe erhaltenen Benzoats (Beispiel 1h) in 495 ml als Methanol wurde mit 3,5 g Kaliumcarbonat (wasserfrei) versetzt und 5 Stunden bei Raumtemperatur unter Argon gerührt. Anschliessend gab man 495 ml 0,1 n Salzsäure zur Reaktionsmischung und rührte weitere 15 Minuten. Nach Einengen der Lösung wurde mit Essigester extrahiert, die vereinigten organischen Phasen wurden dann mit gesättigter Natriumchloridlösung gewaschen, getrocknet und zur Trockne eingeengt. Der Rückstand wurde gegebenenfalls durch Säulenchromatographie an Kieselgel mit Essigester als Laufmittel gereinigt. Man erhielt 7,3 g der gewünschten Verbindung.

IR (Film): 3460, 1765, 1180, 1030, 965/cm.

1j) (1S,5R,6R,7R)-6-[(E)-(3S)-(4RS)-3-Tetrahydropyran-2-yloxy)-4,7-dimethyl-1,6-octadienyl]-7-(tetrahydropyran-2-yloxy)-2-oxabicyclo-[3.3.0]-octan-3-on

Zu einer Lösung von 7,3 g des in der vorigen Reaktionsstufe erhaltenen Diols in 250 ml abs. Methylenchlorid gab man 6,52 ml frisch über Kaliumhydroxid destilliertes Dihydropyran und 1 g Pyridin-p-Toluolsulfonat. Nach 21stündigem Rühren bei Raumtemperatur wurde die Reaktionslösung mit halbgesättigter Natriumchloridlösung gewaschen, über Magnesiumsulfat getrocknet und im Vakuum eingeengt. Der Eindampfrückstand wurde durch Säulenchromatographie an Kieselgel mit Äther als Fliessmittel gereinigt. Es wurden 10,6 g der Titelverbindung erhalten.

IR (Film): 1775, 1180, 1130, 1075, 1020, 970, 810/cm.

1k) (2RS,3aR,4R,5R,6aS)-4-[(E)-(3S)-(4RS)-3-(tetrahydropyran-2-yloxy)-4,7-dimethyl-1,6-octadienyl]-5-(tetrahydropyran-2-yloxy)-perhydrocyclopenta[b]furan-2-ol

Zu einer auf −70 °C gekühlten Lösung der in der vorigen Reaktionsstufe erhaltenen 10,53 g Lakton (Beispiel 1j) in 300 ml abs. Toluol unter Argon wurden 31,2 ml einer 20%igen Diisobutylaluminiumhydrid-Lösung in Toluol innerhalb von 10–15 Minuten getropft. Man rührte 10 Minuten nach, gab dann tropfenweise 2,21 ml Isopropanol und bei 0 °C 16 ml Wasser zu und liess weitere 10 Minuten rühren. Der entstandene weisse körnige Niederschlag wurde über eine Fritte abgetrennt und mit Essigester nachgewaschen. Die organischen Phasen wurden mit gesättigter Natriumchloridlösung dreimal gewaschen, über Magnesiumsulfat getrocknet und im Vakuum eingeengt. Man erhielt 10,4 g Öl, die ohne weitere Reinigung in die nächste Reaktionsstufe eingesetzt wurden.

1l) (5Z,13E)-(8R,9S,11R,12R,15S,16RS)-9-Hydroxy-11,15-bis-(tetrahydropyran-2-yloxy)-16,19-dimethyl-5,13,18-prostatriensäure

225 ml einer Lösung von Methansulfinylmethylnatrium in abs. Dimethylsulfoxid (Lösung: 15 g 50%ige Natriumhydrid-Suspension in 300 ml abs. Dimethylsulfoxid eine halbe Stunde bei 70 °C rühren) wurden zu einer Lösung von 50 g 4-Carboxybutyltriphenylphosphoniumbromid (1,5 Stunden bei 75–80 °C an der Ölpumpe getrocknet) in 200 ml abs. Dimethylsulfoxid bei ca. 15 °C zugetropft. Nach 30 Minuten Rühren bei Raumtemperatur wurde diese Ylenlösung bei 15 °C innerhalb von 15 Minuten zu einer Lösung der in dem vorhergehenden Reaktionsschritt erhaltenen 10,4 g Lactol (Beispiel 1k) in 200 ml abs. Dimethylsulfoxid und 100 ml abs. Tetrahydrofuran getropft. Danach wurde die Reaktionsmischung 7,5 Stunden bei Raumtemperatur gerührt. Anschliessend wurde mit ca. 400 ml Eis/Wasser-Gemisch versetzt und dreimal mit Äther extrahiert. Die wässrige Phase wurde mit 10%iger Citronensäurelösung auf pH = 4 eingestellt und je dreimal mit einer 1/1-Mischung aus Äther/Hexan und Methylenchlorid ausgeschüttelt. Laut analytischer Dünnschichtchromatrographie konnte die Methylenchlorid-Phase verworfen werden. Die anderen organischen Phasen wurden vereinigt, mit gesättigter Natriumchlorid-Lösung dreimal gewaschen, über Natriumsulfat getrocknet und am Rotationsverdampfer eingeengt. Der Rückstand wurde durch Säulenchromatographie an Kieselgel mit Hexan/50–70% Essigester als Fliessmittel gereinigt. Es wurden 8,15 g der gewünschten Carbonsäure erhalten.

IR (Film): 3460 (breit), 2730, 2660, 1735, 1710, 1135, 1080, 1020, 975, 810/cm.

1m) (5Z,13E)-(8R,9S,11R,12R,15S,16RS)-9-Hydroxy-11,15-bis-(tetrahydropyran-2-yloxy)-16,19-dimethyl-5,13,18-prostatriensäure-methylester

8,15 g der nach obiger Vorschrift (Beispiel 1l) erhaltenen Carbonsäure wurden in wenig Methylenchlorid gelöst und mit so viel ätherischer Diazomethanlösung versetzt, bis keine Gasentwicklung mehr auftrat und die Gelbfärbung der Reaktionslösung bestehenblieb. Nach Entfernen des überschüssigen Diazomethans sowie des Lösungsmittels im Vakuum bei Raumtemperatur wurden 8,1 g der Titelverbindung als farbloses Öl erhalten.

IR (Film): 3400 (breit), 1740, 1135, 1080, 1020, 975, 810/cm.

1n) (5Z,13E)-(8R,9S,11R,12R,15S,16RS)-16,19-Dimethyl-11,15-bis-(tetrahydropyran-2-yloxy)-9-toxyloxy-5,13,18-prostatriensäure-methylester

Eine Lösung von 3 g des nach Beispiel 1m) erhaltenen Carbonsäureesters in 5,2 ml trockenem Pyridin wurde bei 0 °C unter Argon mit 2,04 g p-Toluolsulfonylchlorid versetzt und 48 Stunden bei Raumtemperatur gerührt. Nach Zugabe von 2,9 ml

Wasser wurde weitere 2 Stunden bei Raumtemperatur gerührt. Anschliessend wurde die Reaktionslösung mit 800 ml Äther verdünnt, nacheinander mit 20 ml Wasser, zweimal mit je 30 ml eiskalter 5%iger Schwefelsäure, mit 20 ml Wasser, mit 30 ml Natriumbicarbonatlösung und endlich mit Wasser neutral gewaschen, über Magnesiumsulfat getrocknet und im Vakuum zur Trockne eingeengt. Die Reinigung des farblosen Öls erfolgte durch Säulenchromatographie an Kieselgel mit Hexan/20–50% Essigester als Laufmittel. Man erhielt 3,28 g der Titelverbindung.

IR (Film): 2940, 2860, 1740, 1600, 1495, 1450, 1440, 1370, 1175, 1030, 1020, 970/cm.

1o) (5Z,13E)-(8R,9R,11R,12R,15S,16RS)-9-Hydroxy-11,15-bis-(tetrahydropyran-2-yloxy)-16,19-dimethyl-5,13,18-prostatriensäure-methylester

Die in der vorigen Reaktionsstufe nach Beispiel 1n) erhaltenen 3,28 g wurden in 67 ml Dimethylsulfoxid gelöst und mit 6,8 g Kaliumnitrit versetzt. nach 4,5stündigem Rühren bei 65 °C (hierbei färbte sich die Lösung erst grün, dann braun) wurde das Reaktionsgemisch in 400 ml gesättigte Natriumchloridlösung gegossen. Man extrahierte dann fünfmal mit je 250 ml Äther, wusch die vereinigten organischen Phasen mit Wasser neutral, trocknete über Magnesiumsulfat und engte im Vakuum zur Trockne ein. Der Rückstand wurde durch Säulenchromatographie an Kieselgel mit Essigester/Hexan = 1/1 als Fliessmittel gereinigt. Man erhielt 1,25 g der gewünschten Verbindung als farbloses Öl.

IR (Film): 3450, 2940, 2870, 1740, 1455, 1440, 1030, 1020, 970/cm.

1p) (5Z,13E)-(8R,9R,11R,12R,15S,16RS)-16,19-Dimethyl-11,15-bis-(tetrahydropyran-2-yloxy)-9-(p-toluolsulfonyloxy)-5,13,18-prostatriensäure-methylester

Zu einer Lösung von 1,25 g des in der vorigen Reaktionsstufe hergestellten 9β-Alkohols in 2,2 ml trockenem Pyridin wurden bei 0 °C 850 mg p-Toluolsulfonylchlorid gegeben. Anschliessend wurde die Reaktionslösung 22 Stunden bei Raumtemperatur unter Argon gerührt. Nach Verdünnen mit Äther wurde die Reaktionsmischung dann nacheinander mit Wasser, eiskalter 5%iger Schwefelsäure, Wasser, Natriumbicarbonat-Lösung und wieder Wasser gewaschen. Man trocknete über Magnesiumsulfat, dampfte im Vakuum ein und reinigte den Rückstand durch Säulenchromatographie an Kieselgel mit Hexan/30% Essigester als Fliessmittel. Es wurden 1,23 g der Titelverbindung erhalten.

IR (Film): 2940, 2860, 1740, 1600, 1490, 1450, 1440, 1370, 1178, 1035, 1025, 970/cm.

1q) (5Z,13E)-(8R,9R,11R,12R,15S,16RS)-11,15-Dihydroxy-16,19-dimethyl-9-(p-toluolsulfonyloxy)-5,13,18-prostatriensäure-methylester

Die nach Beispiel 1p) erhaltenen 1,23 g Tosylat wurden 21 Stunden mit 36 ml einer Mischung aus Essigsäure/Wasser/Tetrahydrofuran (65/35/10) unter Argon bei Raumtemperatur gerührt. Anschliessend wurde das Reaktionsgemisch mit 200 ml Sole versetzt, fünfmal mit je 150 ml Essigester extrahiert, die organische Phase mit gesättigter Natriumbicarbonatlösung und danach mit Wasser neutral gewaschen, über Natriumsulfat getrocknet und im Vakuum eingeengt. Der Eindampfrückstand wurde einer Säulenchromatographie an Kieselgel mit Essigester/Hexan (2/1) als Fliessmittel unterworfen. Man erhielt 766 mg des Tosylats als farbloses Öl.

IR: 3400, 2950, 2920, 2870, 1735, 1595, 1495, 1450, 1435, 1360, 1175, 1095, 970/cm.

1r) (5Z,13E)-(8R,9S,11R,12R,15S,16RS)-9-Azido-11,15-dihydroxy-16,19-dimethyl-5,13,18-prostatriensäure-methylester

Eine Lösung aus 752 mg des nach Beispiel 1q) hergestellten Diols in 16,5 ml Hexamethylphosphorsäuretriamid versetzte man mit 161 mg Natriumazid und rührte 4,5 Stunden bei 40 °C. Das abgekühlte Reaktionsgemisch wurde mit 100 ml Eiswasser versetzt, fünfmal mit je 50 ml Äther extrahiert, die organische Phase dreimal mit Natriumchlorid-Lösung gewaschen, über Magnesiumsulfat getrocknet und im Vakuum eingeengt. Die so als dünnschichtchromatographisch einheitliches Öl erhaltene Titelverbindung wurde ohne weitere Reinigung in die nächste Reaktion eingesetzt.

IR: 3380, 2960, 2930, 2870, 2100, 1740, 1450, 1435, 970/cm.

1s) (5Z,13E)-(8R,9S,11R,12R,15S,16RS)-9-Azido-11,15-dihydroxy-16,19-dimethyl-5,13,18-prostatriensäure

Das nach Beispiel 1r) erhaltene Azid wurde in 13,7 ml einer Lösung aus 3,6 g Kaliumhydroxid, 24 ml Wasser und 120 ml Methanol gegeben und 4 Stunden bei Raumtemperatur unter Argon gerührt. Das Reaktionsgemisch wurde dann in 40 ml Wasser gegeben und einmal mit Äther/Hexan (1/1) gewaschen, die Wasserphase wurde anschliessend auf 5 °C abgekühlt, mit 10%iger Citronensäure-Lösung auf pH 6 angesäuert und fünfmal mit je 50 ml Methylenchlorid extrahiert. Die vereinigten organischen Phasen wurden mit 100 ml gesättigter Natriumchlorid-Lösung gewaschen, über Magnesiumsulfat getrocknet und im Vakuum eingeengt. Der Eindampfrückstand wurde einer Reinigung durch Säulenchromatographie an Kieselgel mit Essigester/0–5% Methanol als Laufmittel unterworfen. Man erhielt 298 mg der Titelverbindung als farbloses Öl.

IR: 3400 (breit), 2950, 2930, 2875, 2120, 1710, 1450, 1435, 970/cm.

Beispiel 2

(13E)-(11R,15R,16RS)-11,15-Dihydroxy-16,19-dimethyl-9α,6-nitrilo-13,18-prostadiensäure

Eine Lösung von 340 mg (5Z,13E)-(8R,9S,11R,12R,15R,16RS)-9-Azido-11,15-dihydroxy-16,19-dimethyl-5,13,18-prostatriensäure in 32 ml Essigsäureäthylester wurde 26 Stunden bei 80 °C unter Argon gerührt. Anschliessend engte man im Vakuum ein und reinigte den Rückstand durch Säulenchromatographie an Kieselgel mit Essigester/0–40% Methanol als Fliessmittel. Es

wurden 205 mg der Titelverbindung als farbloses Öl erhalten.

IR: 3400 (breit), 2960, 2920, 2870, 1710, 1640, 1085, 1020, 970/cm.

Das Ausgangsmaterial für die obige Titelverbindung wurde wie folgt dargestellt:

2a) (1S,5R,6R,7R)-7-Benzoyloxy-[(E)-(3R,4RS)-3-hydroxy-4,7-dimethyl-1,6-octadienyl]-2-oxabicyclo[3.3.0]-octan-3-on

Bei der Natriumborhydrid-Reduktion des Ketons von Beispiel 1g) wurden neben den 4,5 g 15α-Alkohol (Beispiel 1h) sowie 0,34 g 2β-Gemisch noch 2,3 g der Titelverbindung als polares Produkt isoliert.

IR (Film): 3480 (breit), 1770, 1715, 1600, 1580, 1275, 1180, 710/cm.

2b) (1S,5R,6R,7R)-6-[(E)-(3R)-(4RS)-3-Hydroxy-4,7-dimethyl-1,6-octadienyl]-7-hydroxy-2-oxabicyclo[3.3.0]-octan-3-on

Analog der für die Benzoatspaltung des15α-Hydroxy-Isomeren (Beispiel 1i) angegebenen Vorschrift wurden 4,58 g des in der vorigen Reaktionsstufe erhaltenen Esters mit 1,6 g wasserfreiem Kaliumcarbonat umgesetzt. Man erhielt 3,27 g Diol.

IR (Film): 3460, 1770, 1180, 1035, 965/cm.

2c) (1S,5R,6R,7R)-6-[(E)-(3R)-(4RS)-3-(Tetrahydropyran-2-yloxy)-4,7-dimethyl-1,6-octadienyl]-7-(tetrahydropyran-2-yloxy)-2-oxabicyclo[3.3.0]-octan-3-on

Entsprechend der für die Ätherbildung des 15α-Hydroxy-Isomeren (Beispiel 1j) angegebenen Vorschrift wurden 3,27 g des in der vorigen Stufe erhaltenen Diols mit 2,93 ml Dihydropyran und 455 mg Pyridin-p-Toluolsulfonat umgesetzt. Nach 25stündiger Reaktionszeit und Säulenchromatographie an Kieselgel mit Äther als Laufmittel wurden 4,3 g der Titelverbindung als farbloses Öl erhalten.

IR (Film): 1775, 1180, 1130, 1075, 1020, 970, 810/cm.

2d) (2RS,3aR,4R,5R,6aS)-4-[(E)-(3R)-(4RS)-3-(tetrahydropyran-2-yloxy)-4,7-dimethyl-1,6-octadienyl]-5-(tetrahydropyran-2-yloxy)-perhydrocyclopenta[b]furan-2-ol

Die in der vorigen Reaktionsstufe erhaltenen 4,3 g Lakton wurden analog der für die Darstellung des isomeren 15α-Laktols (Beispiel 1k) angegebenen Vorschrift mit 12,7 ml 20%iger Diisobutylaluminiumhydridlösung umgesetzt. Man erhielt 4,25 g Laktol, die ohne weitere Reinigung in die nächste Stufe eingesetzt wurden.

2e) (5Z,13E)-(8R,9S,11R,12R,15R,16RS)-9-Hydroxy-11,15-bis-(tetrahydropyran-2-yloxy)-16,19-dimethyl-5,13,18-prostatriensäure

Die in der vorigen Reaktionsstufe erhaltenen 4,25 g wurden analog der Vorschrift für die Darstellung des entsprechenden 16α-Isomeren (Beispiel 1l) mit 20,35 g 4-Carboxybutyltriphenylphosphoniumbromid und 88,5 ml Methansulfinylmethylnatrium umgesetzt. Man erhielt 3,2 g Carbonsäure.

IR (Film): 3460 (breit), 2725, 2660, 1735, 1705, 1130, 1080, 1020, 975, 810/cm.

2f) (5Z,13E)-(8R,9S,11R,12R,15R,16RS)-9-Hydroxy-11,15-bis-(tetrahydropyran-2-yloxy)-16,19-dimethyl-5,13,18-prostatriensäure-methylester

Analog der für die Darstellung des 15α-Isomeren (Beispiel 1m) angegebenen Methode wurden die 3,2 g Carbonsäure des Beispiels 2e) umgesetzt. Es wurden 3,15 g Ester erhalten.

IR (Film): 3400 (breit), 1740, 1135, 1080, 1020, 975, 810/cm.

2g) (5Z,13E)-(8R,9S,11R,12R,15R,16RS)-16,19-Dimethyl-11,15-bis-(tetrahydropyran-2-yloxy)-9-tosyloxy-5,13,18-prostatriensäure-methylester

Eine Lösung von 3,15 g des nach Beispiel 2f) erhaltenen Carbonsäureesters in 5,5 ml trockenem Pyridin wurde bei 0 °C und unter Argon mit 2,14 g p-Toluolsulfonylchlorid versetzt und 50 Stunden bei Raumtemperatur gerührt. Nach Zugabe von 3 ml Wasser wurde weitere 2 Stunden bei Raumtemperatur gerührt. Anschliessend wurde die Reaktionslösung mit 800 ml Äther verdünnt, nacheinander mit 20 ml Wasser, zweimal mit je 30 ml eiskalter 5%iger Schwefelsäure, mit 20 ml Wasser, mit 30 ml Natriumbicarbonat-Lösung und endlich mit Wasser neutral gewaschen, über Magnesiumsulfat getrocknet und im Vakuum zur Trockne eingeengt. Die Reinigung des farblosen Öls erfolgte durch Säulenchromatographie an Kieselgel mit Hexan/20–50% Essigester als Laufmittel. Es wurden 3,7 g der Titelverbindung erhalten.

IR (Flüssigkeitsfilm): 2940, 2850, 1740, 1600, 1490, 1450, 1440, 1360, 1170, 1030, 1020, 970/cm.

2h) (5Z,13E)-(8R,9R,11R,12R,15R,16RS)-9-Hydroxy-11,15-bis-(tetrahydropyran-2-yloxy)-16,19-dimethyl-5,13,18-prostatriensäure-methylester

Die in der vorigen Reaktionsstufe (Beispiel 2g) erhaltenen 3,7 g wurden in 75 ml Dimethylsulfoxid gelöst und mit 7,6 g Kaliumnitrit versetzt. Nach 4,5stündigem Rühren bei 65 °C (hierbei färbte sich die Lösung erst grün, dann braun) wurde das Reaktionsgemisch in 400 ml gesättigte Natriumchlorid-Lösung gegossen. Man extrahierte dann fünfmal mit je 250 ml Äther, wusch die vereinigten organischen Phasen mit Wasser neutral, trocknete über Magnesiumsulfat und engte in Vakuum zur Trockne ein. Der Rückstand wurde durch Säulenchromatographie an Kieselgel mit Essigester/Hexan = 1/1 als Laufmittel gereinigt. Es wurden 1,5 g der Titelverbindung als farbloses Öl erhalten.

IR: 3400, 2940, 2860, 1740, 1455, 1440, 1030, 1020, 970/cm.

2i) (5Z,13E)-(8R,9R,11R,12R,15R,16RS)-16,19-Dimethyl-11,15-bis-(tetrahydropyran-2-yloxy)-9-(p-toluolsulfonyloxy)-5,13,18-prostatriensäure-methylester

Zu einer Lösung von 1,5 g des in der vorigen Reaktionsstufe hergestellten 9β-Alkohols in 2,6 ml trockenem Pyridin wurde bei 0 °C 1 g p-Toluolsulfonylchlorid gegeben. Anschliessend wurde die Reaktionslösung 22 Stunden bei Raumtemperatur unter Argon gerührt. Nach Verdünnen mit Äther wurde die Reaktionsmischung dann nacheinander mit Wasser, eiskalter 5%iger Schwefelsäure,

Wasser, Natriumbicarbonat-Lösung und wieder Wasser gewaschen. Man trocknete über Magnesiumsulfat, dampfte im Vakuum ein und reinigte den Rückstand durch Säulenchromatographie an Kieselgel mit Hexan/30% Essigester als Fliessmittel. Man erhielt 1,5 g der Titelverbindung.

IR (Film): 2940, 2860, 1740, 1600, 1495, 1450, 1440, 1370, 1170, 1030, 1020, 970/cm.

2j) (5Z,13E)-(8R,9R,11R,12R,15R,16RS)-11,15-Dihydroxy-16,19-dimethyl-9-(p-toluolsulfonyloxy)-5,13,18-prostatriensäure-methylester

Die nach Beispiel 2i) erhaltenen 1,5 g Toxylat wurden 21 Stunden mit 4,5 ml einer Mischung aus Essigsäure/Wasser/Tetrahydrofuran (65/35/10) unter Argon bei Raumtemperatur gerührt. Anschliessend wurde das Reaktionsgemisch mit 200 ml Sole versetzt, fünfmal mit je 150 ml Essigester extrahiert, die organische Phase mit gesättigter Natriumbicarbonat-Lösung und danach mit Wasser neutral gewaschen, über Natriumsulfat getrocknet und im Vakuum eingeengt. Der Eindampfrückstand wurde einer Säulenchromatographie an Kieselgel mit Essigester/Hexan (2/1) als Fliessmittel unterworfen. Man erhielt 910 mg des Tosylats.

IR (Film): 3400, 2950, 2920, 2870, 1740, 1595, 1490, 1450, 1435, 1360, 1175, 1090, 970/cm.

2k) (5Z,13E)-(8R,9S,11R,12R,15R,16RS)-9-Azido-11,15-dihydroxy-16,19-dimethyl-5,13,18-prostatriensäure-methylester

Eine Lösung aus 900 mg des nach Beispiel 2j) hergestellten Diols in 20 ml Hexamethylphosphorsäuretriamid versetzte man mit 190 mg Natriumazid und rührte 4,5 Stunden bei 40 °C. Das abgekühlte Reaktionsgemisch wurde mit 100 ml Eiswasser versetzt, fünfmal mit je 50 ml Äther extrahiert, die organische Phase dreimal mit Natriumchlorid-Lösung gewaschen, über Magnesiumsulfat getrocknet und im Vakuum eingeengt. Die so als dünnschichtchromatographisch einheitliches Öl erhaltene Titelverbindung wurde ohne weitere Reinigung in die nächste Reaktion eingesetzt.

IR (Film): 3400, 2960, 2930, 2870, 2100, 1740, 1450, 1430, 970/cm.

2l) (5Z,13E)-(8R,9S,11R,12R,15R,16RS)-9-Azido-11,15-dihydroxy-16,19-dimethyl-5,13,18-prostatriensäure

Das nach Beispiel 2k) erhaltene Azid wurde in 16,4 ml einer Lösung aus 3,6 g Kaliumhydroxid, 24 ml Wasser und 120 ml Methanol gegeben und 4 Stunden bei Raumtemperatur unter Argon gerührt. Das Reaktionsgemisch wurde dann in 40 ml Wasser gegeben und einmal mit Äther/Hexan (1/1) gewaschen, die Wasserphase wurde anschliessend auf 5 °C abgekühlt, mit 10%iger Citronensäure-Lösung auf pH 6 angesäuert und fünfmal mit je 50 ml Methylenchlorid extrahiert. Die vereinigten organischen Phasen wurden mit 100 ml gesättigter Natriumchloridlösung gewaschen, über Magnesiumsulfat getrocknet und im Vakuum eingeengt. Der Eindampfrückstand wurde einer Reinigung durch Säulenchromatographie an Kieselgel mit Essigester/0–5% Methanol als Laufmittel unterworfen. Man erhielt 340 mg der Titelverbindung.

IR (Film): 3400 (breit), 2950, 2930, 2870, 2120, 1710, 1450, 1430, 970/cm.

Beispiel 3

(13E)-11R,15S)-11,15-Dihydroxy-16,16,19-trimethyl-9α,6-nitrilo-13,18-prostadiensäure

Eine Lösung von 210 mg (5Z,13E)-(8R,9S,11R,12R,15R)-9-Azido-11,15-dihydroxy-16,16,19-trimethyl-5,13,18-prostatriensäure in 18 ml Essigsäureäthylester wurde 27 Stunden bei 80 °C unter Argon gerührt. Anschliessend dampfte man im Vakuum ein und reinigte den Rückstand durch Säulenchromatographie an Kieselgel mit Essigester/0–50% Methanol als Fliessmittel. Es wurden 130 mg der Titelverbindung als farbloses Öl erhalten.

IR: 3400 (breit), 2960, 2920, 2875, 1705, 1640, 1085, 1020, 975/cm.

Das Ausgangsmaterial für die obige Titelverbindung wurde wie folgt hergestellt:

3a) 2,2,5-Trimethyl-4-hexensäure-äthylester

Eine Lösung von 101,2 g Diisopropylamin in 125 ml abs. Tetrahydrofuran wurde unter Argon bei −20 °C tropfenweise mit 610 ml einer 1,64 n Butyllithium-Lösung in Hexan versetzt. Man liess die Temperatur kurzzeitig auf ca. 0 °C steigen, um dann bei −50 bis −60 °C 116 g Isobuttersäureäthylester tropfenweise zur Lithiumdiisopropylamid-Lösung zu geben. Die Reaktionslösung wurde eine Stunde bei 0 °C gerührt, dann auf −40 °C gekühlt und anschliessend zu einer auf −20 °C gehaltenen Lösung von 200 g 4-Brom-2-methyl-2-buten (Dimethyl-allylbromid) in 60 ml abs. Dimethylsulfoxid gegeben. Unter Ansteigenlassen der Temperatur auf Raumtemperatur wurde das Reaktionsgemisch 60 Stunden gerührt und anschliessend mit 250 ml gesättigter Natriumchlorid-Lösung versetzt. Die organische Phase wurde abgetrennt und die wässrige Phase fünfmal mit je 200 ml eines 1/1-Gemisches aus Äther und Hexan extrahiert. Die vereinigten organischen Phasen wurden mit 0,5 n Salzsäure und gesättigter Natriumchlorid-Lösung neutral gewaschen, über Magnesiumsulfat getrocknet und am Rotationsverdampfer eingeengt. Der Rückstand wurde im Vakuum destilliert. Man erhielt 91,5 g des gewünschten Esters $Kp_{13}$ = 76–81 °C.

IR (Film): 1735, 1160, 1060, 820/cm.

3b) 2,2,5-Trimethyl-4-hexensäure-methylester

Die Darstellung folgte der obigen Vorschrift für die Synthese des entsprechenden Äthylesters.

$Kp_{13}$ = 72–74 °C.

IR (Film): 1735, 1160, 1050, 820/cm.

3c) 2,2,5-Trimethyl-4-hexensäure

Die Darstellung erfolgte nach obiger Vorschrift mit Isobuttersäure als Edukt sowie 2 Äquivalenten Lithiumdiisopropylamid. Kp 0,2–0,4 = 94–100 °C.

IR (Film): 1705, 1220, 820/cm.

3d) 2-(1,1,4-Trimethyl-3-pentenyl)-3-oxoäthanphosphonsäure-dimethylester

Zu einer Lösung von 13 g Methanphosphonsäuredimethylester in 160 ml abs. Tetrahydrofuran tropfte man unter Argon bei −60 °C 49,5 ml einer 2,02 n Butyllithium-Lösung in Hexan. Nach 15 Minuten wurde eine Lösung von 9,2 g 2,2,5-Trimethyl-4-hexensäure-äthylester in 25 ml abs. Tetrahydrofuran zugetropft. Nach 2 Stunden bei −60 °C liess man das Reaktionsgemisch sich innerhalb einer Stunde auf Raumtemperatur erwärmen, versetzte es mit 5,72 ml Eisessig und engte es dann im Vakuum ein. Den Rückstand, eine weisse, gelartige Masse, verteilte man in einem Zweiphasengemisch aus 35 ml Wasser und 165 ml Äther. Die organische Phase wurde über Magnesiumsulfat getrocknet und am Rotationsverdampfer eingeengt. Nach Abdestillieren der flüchtigen Nebenprodukte bei 60 °C und 0,1 Torr reinigte man den Rückstand durch Säulenchromatographie an Kieselgel mit Hexan/50–100% Essigester als Fliessmittel. Man erhielt 8,6 g der Titelverbindung.

IR (Film): 1705, 1260, 1030, 800/cm.

3e) (1S,5R,6R,7R)-7-Benzoyloxy-6-[(E)-4,4,7-trimethyl-3-oxo-1,6-octadienyl]-2-oxabicyclo-[3.3.0]-octan-3-on

Zu einer Suspension von 1,1 g 50%igem (in Öl suspendiertem) Natriumhydrid in 120 ml frisch über Lithiumaluminiumhydrid destilliertem Dimethoxyäthan wurden unter Argon bei Raumtemperatur 6,06 g des in der vorigen Reaktionsstufe erhaltenen Phosphonats (gelöst in 60 ml abs. Dimethoxyäthan) getropft. Nach Zugabe von 0,98 g Lithiumchlorid (vorher im Vakuum 2 Stunden bei 50 °C getrocknet) wurde das Reaktionsgemisch 2 Stunden bei Raumtemperatur gerührt. Anschliessend wurde die Suspension auf −20 °C abgekühlt und tropfenweise mit einer Lösung von 6,34 g (1S,5R,6R,7R)-6-Formyl-7-benzoyloxy-2-oxabicyclo[3.3.0]-octan-3-on [E.J. Corey et al., J. Amer. Chem. Soc. 91, 5675 (1969)] in 180 ml abs. Dimethoxyäthan innerhalb einer halben Stunde versetzt. Danach liess man die Temperatur innerhalb von 2 Stunden unter Rühren auf 0 °C ansteigen. Nach erfolgter analytischer Dünnschichtchromatographie-Kontrolle wurden anschliessend bei −10 °C 2,3 ml Eisessig tropfenweise zugegeben. Danach versetzte man mit 350 ml Wasser, trennte die Phasen, schüttelte die wässrige Phase dreimal mit je ca. 200 ml Äther aus, vereinigte die organischen Phasen und wusch sie mit 4%iger Natriumbicarbonat- und gesättigter Natriumchlorid-Lösung. Nach Trocknen über Magnesiumsulfat wurde das Lösungsmittel am Rotationsverdampfer entfernt. Nach Reinigung des Rückstands durch Säulenchromatographie an Kieselgel mit Hexan/20–40% Essigester als Fliessmittel erhielt man 5,58 g der Titelverbindung als farbloses Öl. Fp.: 98 °C.

IR (KBr): 1775, 1720, 1690, 1625, 1600, 1580, 1275, 1180, 710/cm.

3f) (1S,5R,6R,7R)-7-Benzoyloxy-6-[(E)-(3R)-3-hydroxy-4,4,7-trimethyl-1,6-octadienyl]-2-oxabicyclo-[3.3.0]-octan-3-on

6,9 g Natriumborhydrid wurden portionsweise zu einer auf −40 °C gekühlten Lösung von 11,9 g des in der vorigen Reaktionsstufe erhaltenen Ketons (Beispiel 3e) in einem Gemisch aus 360 ml Methanol und 180 ml Tetrahydrofuran gegeben. Nach 2,5 Stunden wurden – ebenfalls bei −40 °C – 15 ml Eisessig unter starker Schaumentwicklung zur Reaktionslösung getropft. Nach Abziehen des Lösungsmittels am Rotationsverdampfer wurde der Rückstand in Methylenchlorid aufgenommen, mit gesättigter Natriumchloridlösung gewaschen, über Magnesiumsulfat getrocknet und im Vakuum eingeengt. Die Isomerentrennung erfolgte durch Säulenchromatographie an Kieselgel mit Hexan/10–40% Essigester als Fliessmittel. Als unpolarstes Produkt wurden 4,1 g der Titelverbindung isoliert.

IR (KBr): 3500 (breit), 1760, 1750, 1720, 1600, 1580, 1275, 1175, 710/cm.

3g) (1S,5R,6R,7R)-6-[(E)-(3R)-3-Hydroxy-4,4,7-trimethyl-1,6-octadienyl]-7-hydroxy-2-oxabicyclo-[3.3.0]octan-3-on

Eine Lösung von 8,2 g des in der vorigen Reaktionsstufe erhaltenen Benzoats (Beispiel 3f) in 395 ml abs. Methanol wurde mit 2,8 g Kaliumcarbonat (wasserfrei) versetzt und 2,5 Stunden bei Raumtemperatur unter Argon gerührt. Anschliessend gab man 395 ml 0,1 n Salzsäure zur Reaktionsmischung und rührte weitere 15 Minuten. Nach Einengen der Lösung wurde mit Essigester extrahiert, die vereinigten organischen Phasen wurden dann mit gesättigter Natriumchlorid-Lösung gewaschen, getrocknet und zur Trockne eingeengt. Der Rückstand wurde durch Säulenchromatographie an Kieselgel mit Hexan/10–100% Essigester als Fliessmittel gereinigt. Man erhielt 4,75 g der Titelverbindung.

IR (KBr): 3410 (breit), 1760, 1180, 1035, 965/cm.

3h) (1S,5R,6R,7R)-6-[(E)-(3R)-3-(Tetrahydropyran-2-yloxy)-4,4,7-trimethyl-1,6-octadienyl]-7-(tetrahydropyran-2-yloxy)-2-oxabicyclo-[3.3.0]octan-3-on

Zu einer Lösung von 4,75 g des in der vorigen Reaktionsstufe erhaltenen Diols in 250 ml abs. Methylenchlorid gab man bei 0 °C 17 ml frisch über Kaliumhydroxid destilliertes Dihydropyran und 42 mg p-Toluolsulfonsäure. Nach 40minütigem Rühren bei dieser Temperatur wurde die Reaktionslösung dreimal mit gesättigter Natriumhydrogen-carbonat- und anschliessend mit gesättigter Natriumchloridlösung gewaschen, über Magnesiumsulfat getrocknet und im Vakuum eingeengt. Der Eindampfrückstand wurde durch Säulenchromatographie an Kieselgel mit Hexan/50–100% Äther als Fliessmittel gereinigt. Es wurden 6,9 g der gewünschten Verbindung erhalten.

IR (Film): 1780, 1180, 1120, 1080, 1020, 975, 815/cm.

3i) (2RS,3aR,4R,5R,6aS)-4-[(E)-(3R)-3-(tetrahydropyran-2-yloxy)-4,4,7-trimethyl-1,6-octadienyl]-5-(tetrahydropyran-2-yloxy)-perhydrocyclopenta[b]furan-2-ol

Zu einer auf −65 °C gekühlten Lösung der in der vorigen Reaktionsstufe erhaltenen 6,9 g Lakton in 150 ml abs. Toluol unter Argon wurden 20 ml einer 20%igen Diisobutylaluminiumhydrid-Lösung in Toluol innerhalb von 10–15 Minuten getropft. Man rührte 20 Minuten nach, gab dann tropfenweise 1 ml Isopropanol und bei 0 °C 22 ml Wasser zu und liess weitere 10 Minuten rühren. Der entstandene, weisse, körnige Niederschlag wurde über eine Fritte abgetrennt und mit Essigester nachgewaschen. Die organischen Phasen wurden mit gesättigter Natriumchlorid-Lösung dreimal gewaschen, über Magnesiumsulfat getrocknet und im Vakuum eingeengt. Man erhielt 7,1 g Öl, die ohne weitere Reinigung in die nächste Reaktionsstufe eingesetzt wurden.

3j) (5Z,13E)-(8R,9S,11R,12R,15R)-9-Hydroxy-11,15-bis-(tetrahydropyran-2-yloxy)-16,16,19-trimethyl-5,13,18-prostatriensäure

139 ml einer Lösung von Methansulfinylmethylnatrium in abs. Dimethylsulfoxid (Lösung: 10 g 50%ige Natriumhydrid-Suspension in 200 ml abs. Dimethylsulfoxid eine halbe Stunde bei 70 °C rühren) wurden zu einer Lösung von 32 g 4-Carboxybutyltriphenylphosphoniumbromid (1,5 Stunden bei 75–80 °C an der Ölpumpe getrocknet) in 200 ml abs. Dimethylsulfoxid bei ca. 15 °C zugetropft. Nach 30 Minuten Rühren bei Raumtemperatur wurde diese Ylenlösung bei 15 °C innerhalb von 15 Minuten zu einer Lösung der in dem vorhergehenden Reaktionsschritt erhaltenen 7,1 g Lactol in 100 m abs. Dimethylsulfoxid getropft. Danach wurde die Reaktionsmischung 3 Stunden bei 45 °C gerührt. Anschliessend wurde mit 500 ml Eis/Wasser-Gemisch versetzt und dreimal mit Äther extrahiert. Die wässrige Phase wurde mit 10%iger Citronensäure-Lösung auf pH = 4 eingestellt und je dreimal mit einer 1/1-Mischung aus Äther/Hexan und Methylenchlorid ausgeschüttelt. Laut analytischer Dünnschichtchromatographie konnte die Methylenchlorid-Phase verworfen werden. Die anderen organischen Phasen wurden vereinigt, mit gesättigter Natriumchlorid-Lösung dreimal gewaschen, über Natriumsulfat getrocknet und am Rotationsverdampfer eingeengt. Der Rückstand wurde durch Säulenchromatographie an Kieselgel mit Hexen/50–100% Äther als Fliessmittel gereinigt. Man erhielt 1,9 g der gewünschten Carbonsäure.

IR (Film): 3460 (breit), 2720, 2640, 1735, 1705, 1130, 1075, 1020, 970, 805/cm.

3k) (5Z,13E)-(8R,9S,11R,12R,15R)-9-Hydroxy-11,15-bis-(tetrahydropyran-2-yloxy)-16,16,19-trimethyl-5,13,18-prostatriensäure-methylester

1,9 g der nach obiger Vorschrift erhaltenen Carbonsäure wurden in 36 ml Methylenchlorid gelöst und mit so viel ätherischer Diazomethan-Lösung versetzt, bis keine Gasentwicklung mehr auftrat und die Gelbfärbung der Reaktionslösung bestehenblieb. Nach Entfernen des überschüssigen Diazomethans sowie des Lösungsmittels im Vakuum bei Raumtemperatur und Reinigung des Rückstands durch Säulenchromatographie an Kieselgel mit Hexan/50–100% Essigester als Fliessmittel wurden 1,92 g der Titelverbindung als farbloses Öl erhalten.

IR (Film): 3400 (breit), 1735, 1135, 1070, 1020, 970, 810/cm.

3l) (5Z,13E)-(8R,9S,11R,12R,15R)-16,16,19-Trimethyl-11,15-bis-(tetrahydropyran-2-yloxy)-9-tosyloxy-5,13,18-prostatriensäure-methylester

Eine Lösung von 2,3 g des in der vorigen Reaktionsstufe (Beispiel 3k) erhaltenen Carbonsäuremethylesters in 3,9 ml trockenem Pyridin wurde bei 0 °C unter Argon mit 2,5 g p-Toluolsulfonylchlorid versetzt und 50 Stunden bei Raumtemperatur gerührt. Nach Zugabe von 2,2 ml Wasser wurde weitere 2 Stunden bei Raumtemperatur gerührt. Anschliessend wurde die Reaktionslösung mit 600 ml Äther verdünnt, nacheinander mit 20 ml Wasser, zweimal mit je 30 ml eiskalter 5%iger Schwefelsäure, mit 20 ml Wasser, mit 30 ml Natriumbicarbonat-Lösung und dann mit Wasser neutral gewaschen, über Magnesiumsulfat getrocknet und im Vakuum zur Trockne eingeengt. Die Reinigung des farblosen Öls erfolgte durch Säulenchromatographie an Kieselgel mit Hexan/30–50% Essigester als Laufmittel. Man erhielt 2,3 g der Titelverbindung.

IR (Flüssigkeitsfilm): 2940, 2880, 1740, 1600, 1493, 1450, 1440, 1370, 1170, 1030, 1020, 975/cm.

3m) (5Z,13E)-(8R,9R,11R,12R,15R)-9-Hydroxy-11,15-bis-(tetrahydropyran-2-yloxy)-16,16,19-trimethyl-5,13,18-prostatriensäure-methylester

Die in der vorigen Reaktionsstufe nach Beispiel 3l) erhaltenen 2,3 g wurden in 50 ml Dimethylsulfoxid gelöst und mit 4,8 g Kaliumnitrit versetzt. Nach 4,5stündigem Rühren bei 65 °C (hierbei färbte sich die Lösung erst grün, dann braun) wurde das Reaktionsgemisch in 300 ml gesättigte Natriumchlorid-Lösung gegossen. Man extrahierte dann fünfmal mit je 250 ml Äther, wusch die vereinigten organischen Phasen mit Wasser neutral, trocknete über Magnesiumsulfat und engte im Vakuum zur Trockne ein. Der Rückstand wurde durch Säulenchromatographie an Kieselgel mit Essigester/Hexan = 1/1 als Fliessmittel gereinigt. Man erhielt 910 mg der gewünschten Verbindung als farbloses Öl.

IR (Film): 3400, 2950, 2870, 1740, 1450, 1440, 1030, 1020, 975/cm.

3n) (5Z,13E)-(8R,9R,11R,12R,15R)-16,16,19-Trimethyl-11,15-bis-(tetrahydropyran-2-yloxy)-9-(p-toluolsulfonyloxy)-5,13,18-prostatriensäure-methylester

Zu einer Lösung von 910 mg des in der vorigen Reaktionsstufe hergestellten 9β-Alkohols in 1,5 ml trockenem Pyridin wurden bei 0 °C 600 mg p-Toluolsulfonylchlorid gegeben. Danach wurde die Reaktionslösung 21 Stunden bei Raumtemperatur unter Argon gerührt. Nach Verdünnen mit Äther wurde die Reaktionsmischung dann nacheinander mit Wasser, eiskalter 5%iger Schwefelsäure, Wasser, Natriumbicarbonatlösung und wieder Wasser gewaschen. Man trocknete über Magnesiumsulfat, dampfte im Vakuum ein und reinigte den Rückstand durch Säulenchromatographie an Kieselgel mit Hexan/30% Essigester als Fliessmittel. Es wurden 810 mg der Titelverbindung erhalten.

IR: 2960, 2870, 1740, 1600, 1490, 1450, 1440, 1370, 1175, 1030, 1020, 975/cm.

3o) (5Z,13E)-(8R,9R,11R,12R,15R)-11,15-Dihydroxy-16,16,19-trimethyl-9-(p-toluolsulfonyloxy)-5,13,18-prostatriensäure-methylester

Die nach Beispiel 3n) erhaltenen 810 mg 9β-Tosylat wurden 22 Stunden mit 23 ml einer Mischung aus Essigsäure/Wasser/Tetrahydrofuran (65/35/10) unter Argon bei Raumtemperatur gerührt. Anschliessend wurde das Reaktionsgemisch mit 150 ml gesättigter Natriumchlorid-

Lösung versetzt, dreimal mit je 100 ml Essigester extrahiert, die organische Phase mit gesättigter Natriumbicarbonat-Lösung und danach mit Wasser neutral gewaschen, über Natriumsulfat getrocknet und im Vakuum eingeengt. Der Eindampfrückstand wurde einer Säulenchromatographie an Kieselgel mit Essigester/Hexan (2/1) als Fliessmittel unterworfen. Man erhielt 500 mg des Tosylats als farbloses Öl.

IR: 3400 (breit), 2950, 2920, 2860, 1740, 1595, 1495, 1450, 1435, 1360, 1175, 1095, 975/cm.

3p) (5Z,13E)-(8R,9S,11R,12R,15R)-9-Azido-11,15-dihydroxy-16,16,19-trimethyl-5,13,18-prostatriensäure-methylester

Eine Lösung von 500 mg des nach Beispiel 3o) hergestellten Diols in 10,5 ml Hexamethylphosphorsäuretriamid wurde mit 105 mg Natriumazid versetzt und 4,5 Stunden bei 40 °C gerührt. Das abgekühlte Reaktionsgemisch wurde mit 100 ml Eiswasser versetzt, fünfmal mit je 50 ml Äther extrahiert, die organische Phase dreimal mit Natriumchlorid-Lösung gewaschen, über Magnesiumsulfat getrocknet und im Vakuum eingeengt. Die so als dünnschichtchromatographisch einheitliches Öl erhaltene Titelverbindung wurde ohne weitere Reinigung in die nächste Reaktion eingesetzt.

IR: 3400 (breit), 2960, 2930, 2870, 2100, 1735, 1450, 1435, 975/cm.

3q) (5Z,13E)-(8R,9S,11R,12R,15R)-9-Azido-11,15-dihydroxy-16,16,19-trimethyl-5,13,18-prostatriensäure

Das in der vorigen Reaktionsstufe erhaltene Azid (Beispiel 3p) wurde in 8,9 ml einer Lösung aus 3,6 g Kaliumhydroxid, 24 ml Wasser und 120 ml Methanol gegeben und 4 Stunden unter Argon bei Raumtemperatur gerührt. Die Reaktionslösung wurde anschliessend in 40 ml Wasser gegossen und einmal mit Äther/Hexan (1/1) gewaschen, die wässrige Phase wurde dann auf 5 °C abgekühlt, mit 10%iger Citronensäure-Lösung auf pH = 6 angesäuert und fünfmal mit je 50 ml Methylenchlorid extrahiert. Die vereinigten organischen Phasen wurden mit 100 ml gesättigter Natriumchlorid-Lösung gewaschen, über Magnesiumsulfat getrocknet und im Vakuum eingeengt. Der Eindampfrückstand wurde einer Reinigung durch Säulenchromatographie an Kieselgel mit Essigester/0–5% Methanol als Laufmittel unterworfen. Man erhielt 210 mg der Titelverbindung als farbloses Öl.

IR: 3400 (breit), 2950, 2920, 2875, 2120, 1705, 1450, 1430, 975/cm.

Beispiel 4

(13E)-(11R,15S)-11,15-Dihydroxy-19-methyl-9α-6-nitrilo-13,18-prostadiensäure

Eine Lösung von 180 mg (5Z,13E)-(8R,9S,11R,12R,15S)-9-Azido-11,15-dihydroxy-19-methyl-5,13,18-prostatriensäure in 18 ml Essigsäureäthylester wurde 25 Stunden bei 80 °C unter Argon gerührt. Danach dampfte man im Vakuum ein und reinigte den Rückstand durch Säulenchromatographie an Kieselgel mit Essigester/5–50% Methanol als Laufmittel. Es wurden 90 mg der Titelverbindung als viskoses Öl erhalten.

IR: 3400 (breit), 2950, 2930, 2870, 1710, 1640, 1080, 1020, 975/cm.

Das Ausgangsmaterial für die obige Titelverbindung wurde wie folgt hergestellt:

4a) (5Z,13E)-(8R,9S,11R,12R,15S)-19-methyl-11,15-bis-(tetrahydropyran-2-yloxy)-9-tosyloxy-5,13,18-prostatriensäure-methylester

Eine Lösung von 1,65 g (5Z,13E)-(8R,9S,11R,15S)-9-Hydroxy-11,15-bis-(tetrahydropyran-2-yloxy)-19-methyl-5,13,18-prostatriensäure-methylester (hergestellt aus der entsprechenden Säure mit ätherischer Diazomethanlösung) in 2,9 ml trockenem Pyridin wurde bei 0 °C unter Argon mit 1,16 g p-Toluolsulfonylchlorid versetzt und 50 Stunden bei Raumtemperatur gerührt. Nach Zugabe von 1,6 ml Wasser wurde weitere 2 Stunden bei Raumtemperatur gerührt. Anschliessend wurde die Reaktionslösung mit 450 ml Äther verdünnt, nacheinander mit 20 ml Wasser, zweimal mit je 30 ml eiskalter 5%iger Schwefelsäure, mit 20 ml Wasser, mit 30 ml Natriumbicarbonat-Lösung und dann mit Wasser neutral gewaschen, über Magnesiumsulfat getrocknet und im Vakuum zur Trockne eingeengt. Die Reinigung des farblosen Öls erfolgte durch Säulenchromatographie an Kieselgel mit Hexan/30–50% Essigester als Laufmittel. Man erhielt 1,7 g der Titelverbindung.

IR (Flüssigkeitsfilm): 2960, 2880, 1735, 1605, 1493, 1450, 1440, 1370, 1175, 1030, 1020, 975/cm.

4b) (5Z,13E)-(8R,9R,11R,12R,15S)-9-Hydroxy-11,15-bis-(tetrahydropyran-2-yloxy)-19-methyl-5,13,18-prostatriensäure-methylester

Die in der vorigen Reaktionsstufe nach Beispiel 4a) erhaltenen 1,7 g wurden in 36 ml Dimethylsulfoxid gelöst und mit 3,6 g Kaliumnitrit versetzt. Nach 4stündigem Rühren bei 65 °C (hierbei färbte sich die Lösung erst grün, dann braun) wurde das Reaktionsgemisch in 300 ml gesättigte Natriumchloridlösung gegossen. Man extrahierte dann fünfmal mit je 250 ml Äther, wusch die vereinigten organischen Phasen mit Wasser neutral, trocknete über Magnesiumsulfat und engte im Vakuum zur Trockne ein. Der Rückstand wurde durch Säulenchromatographie an Kieselgel mit Essigester/Hexan = 1/1 als Fliessmittel gereinigt. Man erhielt 700 mg der gewünschten Verbindung als farbloses Öl.

IR: 3400, 2960, 2870, 1735, 1450, 1440, 1030, 1020, 975/cm.

4c) (5Z,13E)-(8R,9R,11R,12R,15S)-19-Methyl-11,15-bis-(tetrahydropyran-2-yloxy)-9-(p-toluolsulfonyloxy)-5,13,18-prostatriensäure-methylester

Zu einer Lösung von 700 mg des in der vorigen Reaktionsstufe hergestellten 9β-Alkohols in 1,3 ml trockenem Pyridin wurden bei 0 °C 500 mg p-Toluolsulfonylchlorid gegeben. Danach wurde die Reaktionslösung 20 Stunden bei Raumtemperatur unter Argon gerührt. Nach Verdünnen mit Äther wurde die Reaktionsmischung dann nacheinander mit Wasser, eiskalter 5%iger Schwefelsäure, Wasser, Natriumbicarbonat-Lösung und wieder Wasser gewaschen. Man trocknete über Magnesiumsulfat, dampfte im Vakuum ein und reinigte den Rückstand durch Säulenchromatographie an Kieselgel mit Hexan/20% Essigester als Fliessmittel. Es wurden 700 mg der Titelverbindung erhalten.

IR: 2960, 2870, 1735, 1600, 1490, 1450, 1440, 1370, 1175, 1030, 1020, 975/cm.

4d) (5Z,13E)-(8R,9R,11R,12R,15S)-11,15-Dihydroxy-19-methyl-9-(p-toluolsulfonyloxy)-5,13,18-prostatriensäure-methylester

Die nach Beispiel 4c) erhaltenen 700 mg 9β-Tosylat wurden 20 Stunden mit 21 ml einer Mischung aus Essigsäure/Wasser/Tetrahydrofuran (65/35/10) unter Argon bei Raumtemperatur gerührt. Anschliessend wurde das Reaktionsgemisch mit 150 ml gesättigter Natriumchlorid-Lösung versetzt, dreimal mit je 100 ml Essigester extrahiert, die organische Phase mit gesättigter Natriumbicarbonat-Lösung und danach mit Wasser neutral gewaschen, über Natriumsulfat getrocknet und im Vakuum eingeengt. Der Eindampfrückstand wurde einer Säulenchromatographie an Kieselgel mit Essigester/Hexan (2/1) als Fliessmittel unterworfen. Man erhielt 460 mg des Tosylats als farbloses Öl.

IR: 3400 (breit), 2950, 2920, 2870, 1735, 1600, 1495, 1450, 1435, 1360, 1175, 1095, 975/cm.

4e) (5Z,13E)-(8R,9S,11R,12R,15S)-9-Azido-11,15-dihydroxy-19-methyl-5,13,18-prostatriensäure-methylester

Eine Lösung von 460 mg des nach Beispiel 4d) hergestellten Diols in 10 ml Hexamethylphosphorsäuretriamid wurde mit 100 mg Natriumazid versetzt und 4 Stunden bei 40 °C gerührt. Das abgekühlte Reaktionsgemisch wurde mit 100 ml Eiswasser versetzt, fünfmal mit je 50 ml Äther extrahiert, die organische Phase dreimal mit Natriumchlorid-Lösung gewaschen, über Magnesiumsulfat getrocknet und im Vakuum eingeengt. Die so als dünnschichtchromatographisch einheitliches Öl erhaltene Titelverbindung wurde ohne weitere Reinigung in die nächste Reaktion eingesetzt.

IR: 3400 (breit), 2960, 2920, 2870, 2100, 1735, 1450, 1435, 975/cm.

4f) (5Z,13E)-(8R,9S,11R,12R,15S)-9-Azido-11,15-dihydroxy-19-methyl-5,13,18-prostatriensäure

Das in der vorigen Reaktionsstufe erhaltene Azid (Beispiel 4e) wurde in 8,4 ml einer Lösung aus 3,6 g Kaliumhydroxid, 24 ml Wasser und 120 ml Methanol gegeben und 4 Stunden unter Argon bei Raumtemperatur gerührt. Die Reaktionslösung wurde anschliessend in 40 ml Wasser gegossen und einmal mit Äther/Hexan (1/1) gewaschen, die wässrige Phase wurde dann auf 5 °C abgekühlt, mit 10%iger Citronensäure-Lösung auf pH 6 angesäuert und fünfmal mit je 50 ml Methylenchlorid extrahiert. Die vereinigten organischen Phasen wurden mit 100 ml gesättigter Natriumchloridlösung gewaschen, über Magnesiumsulfat getrocknet und im Vakuum eingeengt. Der Eindampfrückstand wurde einer Reinigung durch Säulenchromatographie an Kieselgel mit Essigester/0–5% Methanol als Laufmittel unterworfen. Man erhielt 180 mg der Titelverbindung als farbloses Öl.

IR: 3400 (breit), 2960, 1920, 1875, 2110, 1710, 1450, 1430, 975/cm.

Beispiel 5

(13E,18Z)-(11R,15S,16RS)-11,15-Dihydroxy-19-chlor-16-methyl-9α,6-nitrilo-13,18-prostadiensäure

Eine Lösung von 190 mg (5Z,13E,18Z)-(8R,9S,11R,12R,15S,16RS)-9-Azido-11,15-dihydroxy-19-chlor-16-methyl-5,13,18-prostatriensäure in 18 ml Essigsäureäthylester wurde 26 Stunden bei 80 °C unter Argon gerührt. Danach engte man im Vakuum zur Trockne ein und reinigte den Rückstand durch Säulenchromatographie an Kieselgel mit Essigester/5–50% Methanol als Fliessmittel. Es wurden 90 mg der Titelverbindung als farbloses Öl erhalten.

IR (Film): 3400 (breit), 1710, 1665, 1635, 1085, 1020, 970/cm.

Das Ausgangsmaterial für die obige Titelverbindung wurde wie folgt hergestellt:

5a) 2-[(2Z)-3-Chlor-2-butenyl]-2-methyl-malonsäurediäthylester

11,5 g in kleine Stücke geschnittenes Natrium wurden in einem mit Rührer, Rückflusskühler und Tropftrichter versehenen Dreihalskolben gegeben. Hierzu wurden 250 ml abs. Äthanol so schnell zugetropft, dass die Lösung lebhaft siedete. Anschliessend wurden 87 g destillierter Methylmalonsäureäthylester zur heissen Alkoholat-Lösung getropft. Nach Abkühlenlassen auf ca. 75 °C wurde die Reaktionslösung unter auftretender Gelbfärbung mit 66 g 1,3-Dichlor-buten-(2) tropfenweise versetzt. Nach 2 Stunden Rühren unter Erhitzen wurde die einen pH-Wert von 5–6 aufweisende und jetzt fast vollständig entfärbte Suspension vom ausgefallenen Natriumchlorid durch Filtration befreit. Das Filtrat wurde eingeengt und mit dem durch Waschen des Niederschlags anfallenden Methylenchlorid vereinigt. Die organische Lösung wurde dann mit gesättigter Natriumchlorid-Lösung geschüttelt, über Magnesiumsulfat getrocknet, am Rotationsverdampfer eingeengt und im Vakuum fraktioniert. Man erhielt 105 g des gewünschten Diesters. $Kp_{2,5}$ = 110 °C.

IR (Film): 1738, 1666, 1160, 1050/cm.

5b) 2-[(2Z)-3-Chlor-2-butenyl]-2-methylmalonsäure

46 g des in der vorigen Reaktionsstufe erhaltenen Diesters wurden zusammen mit 33 g Kaliumhydroxid in 85 ml Äthanol und 45 ml Wasser 3,5 Stunden unter Rückfluss erhitzt. Nach Abziehen des Lösungsmittels im Vakuum wurde der Rückstand in 45 ml Wasser aufgenommen und unter Eiskühlung tropfenweise mit konz. Salzsäure bis zum pH-Wert 1 angesäuert. Anschliessend wurde die wässrige Phase fünfmal mit je 200 ml Äther ausgeschüttelt. Die vereinigten Äther-Extrakte wurden mit gesättigter Natriumchlorid-Lösung gewaschen, über Magnesiumsulfat getrocknet und im Vakuum zur Trockne eingeengt. Der Rückstand wurde aus Benzol/Cyclohexan umkristallisiert. Man erhielt 33,5 g der Disäure. Fp.: 99–101 °C.

IR (KBr): 2700, 2650, 2580, 1700, 1663, 1238/cm.

5c) (4Z)-5-Chlor-2-methyl-4-hexensäure

33,5 g der in der vorigen Reaktionsstufe erhaltenen Dicarbonsäure wurden 4 Stunden auf 160 °C unter $CO_2$-Entwicklung erhitzt. Das Produkt wurde anschliessend im Vakuum destilliert. Man erhielt 24,3 g der Monocarbonsäure. $Kp_{13}$ = 133–135 °C.

IR (Film): 2660, 2570, 1710, 1668, 1243/cm.

5d) (4Z)-5-Chlor-2-methyl-4-hexensäuremethylester

Zu einer Lösung von 24,3 g der nach obiger Vorschrift erhaltenen Carbonsäure in 450 ml Acetonitril wurden nacheinander 153 ml N-Äthyldiisopropylamin und 307 ml Jodmethan getropft. Nach 4stündigem Rühren bei Raumtemperatur wurde die Reaktionslösung mit eiskalter gesättigter Natriumchlorid-Lösung versetzt und mit Essigester extrahiert. Die vereinigten organischen Phasen wurden nacheinander mit Natriumbisulfat, Natriumhydrogencarbonat und gesättigter Natriumchlorid-Lösung gewaschen, über Natriumsulfat getrocknet und am Rotationsverdampfer eingeengt. Der Rückstand wurde im Vakuum destilliert. Es wurden 21,9 g des gewünschten Esters isoliert. $Kp_{13}$ = 81–83 °C.

IR (Film): 1738, 1665, 1195, 1172/cm.

5e) 2-[(3Z)-4-Chlor-1-methyl-3-pentenyl]-2-oxoäthanphosphonsäuredimethylester

Zu einer Lösung von 67,1 g Methanphosphonsäuredimethylester in 840 ml abs. Tetrahydrofuran tropfte man unter Argon bei −60 °C 247,5 ml einer 2,02 m Butyllithium-Lösung in Hexan. Nach 15 Minuten wurde eine Lösung von 44,16 g des nach obiger Vorschrift erhaltenen Esters in 100 ml abs. Tetrahydrofuran zugetropft. Man hielt das Reaktionsgemisch 3,5 Stunden bei −65 °C, über Nacht bei −32 °C, und liess es schliesslich sich auf Raumtemperatur erwärmen. Danach wurde es mit 28,6 ml Eisessig versetzt und im Vakuum zur Trockne eingeengt. Der Rückstand wurde in einem Zweiphasensystem aus 175 ml Wasser und 825 ml Äther verteilt, die organische Phase über Magnesiumsulfat getrocknet und am Rotationsverdampfer eingeengt. Der Eindampfrückstand wurde durch Destillation bei 40 °C/0,1 mm von flüchtigen Nebenprodukten und unumgesetztem Edukt befreit und anschliessend durch Säulenchromatographie an Kieselgel mit Hexan/50–100% Essigester als Fliessmittel gereinigt. Neben 13,8 g Edukt erhielt man 36 g Phosphonat.

IR (Film): 1712, 1666, 1260, 1032/cm.

5f) (1S,5R,6R,7R)-7-Benzoyloxy-6-[(1E,6Z)-(4RS)-7-chlor-4-methyl-3-oxo-1,6-octadienyl]-2-oxabicyclo[3.3.0]-octan-3-on

Zu einer Suspension von 0,96 g 50%igem (in Öl suspendiertem) Natriumhydrid in 90 ml frisch über Lithiumaluminiumhydrid destilliertem Dimethoxyäthan wurden unter Argon bei Raumtemperatur 5,4 g des in der vorigen Reaktionsstufe erhaltenen Phosphonats (gelöst in 60 ml abs. Dimethoxyäthan) getropft. Nach Zugabe von 0,9 g Lithiumchlorid (vorher im Vakuum 2 Stunden bei 50 °C getrocknet) wurde das Reaktionsgemisch 1,5 Stunden bei Raumtemperatur gerührt. Anschliessend wurde die Suspension auf −20 °C abgekühlt und tropfenweise mit einer Lösung von 5,5 g (1S,5R,6R,7R)-6-Formyl-7-benzoyloxy-2-oxabicyclo[3.3.0]octan-3-on [E.J. Corey et al., J. Amer. Chem. Soc. 91, 5675 (1969)] in 150 ml abs. Dimethoxyäthan innerhalb einer halben Stunde versetzt. Danach liess man die Temperatur innerhalb von 2 Stunden unter Rühren auf 0 °C ansteigen. Nach erfolgter analytischer Dünnschichtchromatographie-Kontrolle wurden anschliessend bei −10 °C 2 ml Eisessig tropfenweise zugegeben. Danach versetzte man mit 250 ml Wasser, trennte die Phasen, schüttelte die wässrige Phase dreimal mit je ca. 200 ml Äther aus, vereinigte die organischen Phasen und wusch sie mit 4%iger Natriumbicarbonat- und gesättigter Natriumchlorid-Lösung. Nach Trocknen über Magnesiumsulfat wurde das Lösungsmittel am Rotationsverdampfer entfernt. Der Rückstand wurde durch Säulenchromatographie an Kieselgel mit Hexan/10–50% Essigester als Fliessmittel gereinigt. Man erhielt 7,2 g des gewünschten Ketons.

IR (Film): 1775, 1720, 1670, 1630, 1600, 1582, 1275, 715/cm.

5g) (1S,5R,6R,7R)-7-Benzoyloxy-6-[(1E,6Z)-(3S,4RS)-7-chlor-3-hydroxy-4-methyl-1,6-octadienyl]-2-oxabicyclo-[3.3.0]octan-3-on

Zu einer auf −40 °C gekühlten Lösung von 7,2 g des in der vorigen Reaktionsstufe erhaltenen Ketons in 250 ml abs. Methanol wurden portionsweise 4 g Natriumborhydrid gegeben. Nach 30 Minuten Nachrühren bei dieser Temperatur wurden – ebenfalls bei −40 °C – 8,87 ml Eisessig zur Reaktionslösung getropft. Nach Entfernen des Lösungsmittels am Rotationsverdampfer wurde der Rückstand mit einem Zweiphasengemisch aus 200 ml Wasser und 300 ml Methylenchlorid versetzt, die abgetrennte Wasserphase mit festem Natriumchlorid versetzt und zweimal mit je ca. 200 ml Methylenchlorid extrahiert. Die vereinigten organischen Phasen wurden mit gesättigter Natriumchlorid-Lösung gewaschen, über Magnesiumsulfat getrocknet und im Vakuum eingeengt. Die Isomerentrennung des Rückstands erfolgte durch Säulenchromatographie an Kieselgel mit Hexan/10–60% Essigester als Fliessmittel. Als unpolarstes Produkt wurden 3,3 g der Titelverbindung isoliert.

IR (Film): 3480 (breit), 1772, 1715, 1667, 1600, 1580, 1273, 1172, 713/cm.

5h) (1S,5R,6R,7R)-6-[(1E,6Z)-(3S)-(4SR)-7-Chlor-3-hydroxy-4-methyl-1,6-octadienyl]-7-hydroxy-2-oxabicyclo-[3.3.0]-octan-3-on

Eine Lösung von 7,1 g des in der vorigen Reaktionsstufe erhaltenen Benzoats (Beispiel 3g) in 335 ml abs. Methanol wurde mit 2,4 g Kaliumcarbonat (wasserfrei) versetzt und 4 Stunden bei Raumtemperatur unter Argon gerührt. Anschliessend gab man 335 ml 0,1 n Salzsäure zur Reaktionsmischung und rührte weitere 15 Minuten. Nach Einengen der Lösung wurde mit Essigester extrahiert, die vereinigten organischen Phasen wurden dann mit gesättigter Natriumchlor-Lösung gewaschen, getrocknet und zur Trockne eingeengt. Der Rückstand wurde durch Säulenchromatographie an Kieselgel mit Hexan/50–100% Essigester als

Laufmittel gereinigt. Man erhielt 4,3 g der Titelverbindung.

IR (Film): 3460, 1770, 1665, 1175, 1035/cm.

5i) (1S,5R,6R,7R)-6-[(1E,6Z)-(3S)-(4RS)-7-Chlor-3-(tetrahydropyran-2-yloxy)-4-methyl-1,6-octadienyl]-7-(tetrahydropyran-2-yloxy)-2-oxabicyclo[3.3.0]octan-3-on

Zu einer Lösung von 4,3 g des in der vorigen Reaktionsstufe erhaltenen Diols in 140 ml abs. Methylenchlorid gab man 3,6 ml frisch über Kaliumhydroxid destilliertes Dihydropyran und 0,55 g Pyridin-p-Toluolsulfonat. Nach 20stündigem Rühren bei Raumtemperatur wurde die Reaktionslösung mit halbgesättigter Natriumchlorid-Lösung gewaschen, über Magnesiumsulfat getrocknet und im Vakuum eingeengt. Der Eindampfrückstand wurde durch Säulenchromatographie an Kieselgel mit Äther als Fliessmittel gereinigt. Es wurden 6,2 g der Titelverbindung erhalten.

IR (Film): 1775, 1665, 1180, 1125, 1080, 1025, 975, 810/cm.

5j) (2RS,3aR,4R,5R,6aS)-4-[(1E,6Z)-(3S)-(4RS)-7-Chlor-3-(tetrahydropyran-2-yloxy)-4-methyl-1,6-octadienyl]-5-(tetrahydropyran-2-yloxy)-perhydrocyclopenta[b]furan-2-ol

Zu einer auf −70 °C gekühlten Lösung der in der vorigen Reaktionsstufe erhaltenen 6,2 g Lakton in 170 ml abs. Toluol unter Argon wurden 17,6 ml einer 20%igen Diisobutylaluminiumhydrid-Lösung in Toluol innerhalb von 10–15 Minuten getropft. Man rührte 10 Minuten nach, gab dann tropfenweise 1,3 ml Isopropanol und bei 0 °C 10 ml Wasser zu und liess weitere 10 Minuten rühren. Der entstandene weisse, körnige Niederschlag wurde über eine Fritte abgetrennt und mit Essigester nachgewaschen. Die organischen Phasen wurden mit gesättigter Natriumchlorid-Lösung dreimal gewaschen, über Magnesiumsulfat getrocknet und im Vakuum eingeengt. Man erhielt 6,2 g Öl, die ohne weitere Reinigung in die nächste Reaktionsstufe eingesetzt wurden.

5k) (5Z,13E,18Z)-(8R,9S,11R,12R,15S,16RS)-19-Chlor-9-hydroxy-11,15-bis-(tetrahydropyran-2-yloxy)-16-methyl-5,13,18-prostatriensäure

Eine Lösung von 13,5 g Kalium-tert.-butylat in 240 ml abs. Dimethylsulfoxid (die 15 Minuten lang auf 70 °C erhitzt worden war) wurde zu einer Lösung von 17 g 4-Carboxybutyltriphenylphosphoniumbromid (1,5 Stunden bei 75–80 °C an der Ölpumpe getrocknet) in 70 ml abs. Dimethylsulfoxid bei ca. 15 °C zugetropft. Nach 30 Minuten Rühren bei Raumtemperatur wurde diese Ylenlösung bei 15 °C innerhalb von 15 Minuten zu einer Lösung der in dem vorhergehenden Reaktionsschritt erhaltenen 6,2 g Lactol in 100 ml abs. Dimethylsulfoxid getropft. Danach wurde die Reaktionsmischung 5,5 Stunden bei Raumtemperatur gerührt. Anschliessend wurde mit ca. 300 ml Eis/Wasser-Gemisch versetzt und dreimal mit Äther extrahiert. Die wässrige Phase wurde mit 10%iger Citronensäurelösung auf pH-4 eingestellt und je dreimal mit einer 1/1-Mischung aus Äther/Hexan und Methylenchlorid ausgeschüttelt. Laut analytischer Dünnschichtchromatographie konnte die Methylenchlorid-Phase verworfen werden. Die anderen organischen Phasen wurden vereinigt, mit gesättigter Natriumchlorid-Lösung dreimal gewaschen, über Natriumsulfat getrocknet und am Rotationsverdampfer eingeengt. Der Rückstand wurde durch Säulenchromatographie an Kieselgel mit Hexan/20–100% Essigester als Fliessmittel gereinigt. Man erhielt 4 g der gewünschten Carbonsäure.

IR (KBr): 2730, 2650, 1725, 1700, 1660, 1130, 1072, 1018, 970/cm.

5l) (5Z,13E,18Z)-(8R,9S,11R,12R,15S,16RS)-19-Chlor-9-hydroxy-11,15-bis-(tetrahydropyran-2-yloxy)-16-methyl-5,13,18-prostatriensäure-methylester

4 g der nach obiger Vorschrift erhaltenen Carbonsäure wurden in wenig Methylenchlorid gelöst und mit so viel ätherischer Diazomethanlösung versetzt, bis keine Gasentwicklung mehr auftrat und die Gelbfärbung der Reaktionslösung bestehenblieb. Nach Entfernen des überschüssigen Diazomethans sowie des Lösungsmittels im Vakuum bei Raumtemperatur wurden 4 g der Titelverbindung als farbloses Öl erhalten.

IR (Film): 3400 (breit), 2930, 2870, 1740, 1665, 1135, 1080, 1020, 970/cm.

5m) (5Z,13E,18Z)-(8R,9S,11R,12R,15S,16RS)-19-Chlor-16-methyl-11,15-bis-(tetrahydropyran-2-yloxy)-9-tosyloxy-5,13,18-prostatriensäure-methylester

Eine Lösung von 4 g des in der vorigen Reaktionsstufe (Beispiel 5l) erhaltenen Carbonsäureesters in 6,7 ml trockenem Pyridin wurde bei 0 °C und unter Argon mit 2,64 g p-Toluolsulfonylchlorid versetzt und 50 Stunden bei Raumtemperatur gerührt. Nach Zugabe von 3,8 ml Wasser wurde weitere 2 Stunden bei Raumtemperatur gerührt. Anschliessend wurde die Reaktionslösung mit 1 l Äther verdünnt, nacheinander mit Wasser, zweimal mit je 30 ml eiskalter 5%iger Schwefelsäure, mit Wasser, mit 30 ml Natriumcarbonat-Lösung und dann mit Wasser neutral gewaschen, über Magnesiumsulfat getrocknet und im Vakuum zur Trockne eingeengt. Die Reinigung des farblosen Öls erfolgte durch Säulenchromatographie an Kieselgel mit Hexan/30–50% Essigester als Laufmittel. Man erhielt 4,1 g der Titelverbindung.

IR (Flüssigkeitsfilm): 2940, 2880, 1740, 1660, 1600, 1495, 1370, 1170, 1030, 1020, 975/cm.

5n) (5Z,13E,18Z)-(8R,9R,11R,12R,15S,16RS)-19-Chlor-9-hydroxy-11,15-bis-(tetrahydropyran-2-yloxy)-16-methyl-5,13,18-prostatriensäure-methylester

Die in der vorigen Reaktionsstufe nach Beispiel 5m) erhaltenen 4,1 g wurden in 85 ml Dimethylsulfoxid gelöst und mit 8,4 g Kaliumnitrit versetzt. Nach 4,5stündigem Rühren bei 65 °C (hierbei färbte sich die Lösung erst grün, dann braun) wurde das Reaktionsgemisch in 400 ml gesättigte Natriumchlorid-Lösung gegossen. Man extrahierte dann fünfmal mit je 250 ml Äther, wusch die vereinigten organischen Phasen mit Wasser neutral, trocknete über Magnesiumsulfat und engte im Vakuum zur Trockne ein. Der Rückstand wurde durch Säulenchromatographie an Kieselgel mit Essigester/Hexan = 1/1 als Fliessmittel gereinigt. Man

erhielt 1,62 g der gewünschten Verbindung als farbloses Öl.

IR (Film): 3400 (breit), 1735, 1665, 1130, 1070, 1020, 970/cm.

5o) (5Z,13E,18Z)-(8R,9R,11R,12R,15S,16RS)-19-Chlor-16-methyl-11,15-bis-(tetrahydropyran-2-yloxy)-9-(p-toluolsulfonyloxy)-5,13,18-prostatriensäure-methylester

Zu einer Lösung von 1,62 g des in der vorigen Reaktionsstufe hergestellten 9β-Alkohols in 2,8 ml trockenem Pyridin wurden bei 0 °C 1 g p-Toluolsulfonylchlorid gegeben. Danach wurde die Reaktionslösung 20 Stunden bei Raumtemperatur unter Argon gerührt. Nach Verdünnen mit Äther wurde die Reaktionsmischung dann nacheinander mit Wasser, eiskalter 5%iger Schwefelsäure, Wasser, Natriumbicarbonat-Lösung und wieder Wasser gewaschen. Man trocknete über Magnesiumsulfat, dampfte im Vakuum ein und reinigte den Rückstand durch Säulenchromatographie an Kieselgel mit Hexan/30% Essigester als Fliessmittel. Es wurden 1,64 g der Titelverbindung erhalten.

IR (Film): 2960, 2870, 1740, 1660, 1600, 1490, 1370, 1175, 1030, 1020, 975/cm.

5p) (5Z,13E,18Z)-(8R,9R,11R,12R,15S,16RS)-19-Chlor-11,15-dihydroxy-16-methyl-9-(p-toluolsulfonyloxy)-5,13,18-prostatriensäure-methylester

Die nach Beispiel 5o) erhaltenen 1,64 g 9β-Tosylat wurden 20 Stunden mit 47 ml einer Mischung aus Essigsäure/Wasser/Tetrahydrofuran (65/35/10) unter Argon bei Raumtemperatur gerührt. Anschliessend wurde das Reaktionsgemisch mit 250 ml gesättigter Natriumchlorid-Lösung versetzt, dreimal mit je 100 ml Essigester extrahiert, die organische Phase mit gesättigter Natriumbicarbonat-Lösung und danach mit Wasser neutral gewaschen, über Natriumsulfat getrocknet und im Vakuum eingeengt. Der Eindampfrückstand wurde einer Säulenchromatographie an Kieselgel mit Essigester/Hexan (2/1) als Fliessmittel unterworfen. Man erhielt 1,0 g des Tosylats als farbloses Öl.

IR (Film): 3400 (breit), 2950, 2920, 2860, 1740, 1665, 1590, 1495, 1360, 1175, 1095, 970/cm.

5q) (5Z,13E,18Z)-(8R,9S,11R,12R,15S,16RS)-9-Azido-19-Chlor-11,15-dihydroxy-16-methyl-5,13,18-prostatriensäure-methylester

Eine Lösung von 1,0 g des nach Beispiel 5p) hergestellten Diols in 21 ml Hexamethylphosphorsäuretriamid wurde mit 206 mg Natriumazid versetzt und 4,5 Stunden bei 40 °C gerührt. Das abgekühlte Reaktionsgemisch wurde mit 100 ml Eiswasser versetzt, fünfmal mit je 50 ml Äther extrahiert, die organische Phase dreimal mit Natriumchlorid-Lösung gewaschen, über Magnesiumsulfat getrocknet und im Vakuum eingeengt. Die so als dünnschichtchromatographisch einheitliches Öl erhaltene Titelverbindung wurde ohne weitere Reinigung in die nächste Reaktion eingesetzt.

IR (Film): 3400 (breit), 2960, 2930, 2870, 2100, 1735, 1663, 975/cm

5r) (5Z,13E,18Z)-(8R,9S,11R,12R,15S,16RS)-9-Azido-19-chlor-11,15-dihydroxy-16-methyl-5,13,18-prostatriensäure

Das in der vorigen Reaktionsstufe erhaltene Azid (Beispiel 5q) wurde in 17,5 ml einer Lösung aus 3,6 g Kaliumhydroxid, 24 ml Wasser und 120 ml Methanol gegeben und 4 Stunden unter Argon bei Raumtemperatur gerührt. Die Reaktionslösung wurde anschliessend in 40 ml Wasser gegossen und einmal mit Äther/Hexan (1/1) gewaschen, die wässrige Phase wurde dann auf 5 °C abgekühlt, mit 10%iger Citronensäure-Lösung auf pH 6 angesäuert und fünfmal mit je 50 ml Methylenchlorid extrahiert. Die vereinigten organischen Phasen wurden mit 100 ml gesättigter Natriumchlorid-Lösung gewaschen, über Magnesiumsulfat getrocknet und im Vakuum eingeengt. Der Eindampfrückstand wurde einer Reinigung durch Säulenchromatographie an Kieselgel mit Essigester/0–5% Methanol als Laufmittel unterworfen. Man erhielt 190 mg der Titelverbindung als farbloses Öl.

IR (Film): 3400 (breit), 2950, 2920, 2875, 2110, 1705, 1660, 975/cm.

Beispiel 6

(13E)-(11R,15S)-11,15-Dihydroxy-16-(2-cyclohexenyl)-17,18,19,20-tetranor-9α,6-nitrilo-13-prostensäure

Eine Lösung von 240 mg (5Z,13E)-(8R,9S,11R,12R,15S)-9-Azido-11,15-dihydroxy-16-(2-cyclohexenyl)-17,18,19,20-tetranor-5,13-prostadiensäure in 23 ml Essigsäureäthylester wurde 26 Stunden bei 80 °C unter Argon gerührt. Anschliessend wurde im Vakuum eingeengt und der Rückstand durch Säulenchromatographie an Kieselgel mit Essigester/3–50% Methanol als Fliessmittel gereinigt. Man erhielt 110 mg der Titelverbindung als farbloses Öl.

IR (Film): 3400 (breit), 2960, 2920, 2865, 1705, 1640, 1080, 1020, 975/cm.

Das Ausgangsmaterial für die obige Titelverbindung wurde wie folgt hergestellt:

6a) 2-(2-Cyclohexenyl)-malonsäure-diäthylester

In einem mit Tropftrichter, Rückflusskühler und Rührer versehenen 2 l-Dreihalskolben wurden innerhalb von 2 Stunden 1,1 l Äthanol zu 69 g feingeschnittenes Natrium getropft. Zu der heissen Alkoholat-Lösung wurden – wieder innerhalb von 2 Stunden – 196 g Malonsäurediäthylester tropfenweise zugegeben. Nach Abkühlenlassen wurden – innerhalb von 2 Stunden – 296 g trans-1,2-Dibromcyclohexan zu der Reaktionslösung gegeben. Danach wurde über Nacht am Rückfluss gekocht. Nach Einengen der Reaktionslösung am Rotationsverdampfer wurde der Rückstand mit Äther und verdünnter Salzsäure versetzt, die organische Phase dann mit gesättigter Natriumchlorid-Lösung gewaschen, eingeengt und im Vakuum destilliert. Man erhielt 197 g vom $Kp_1$ = 119–121 °C.

IR (Film): 1750, 1735, 1650 (schwach), 1180, 1035/cm.

6b) (2-Cyclohexenyl)-essigsäure

100 g 2-(2-Cyclohexenyl)-malonsäure-diäthylester und 52 g Kaliumhydroxid in 100 ml Wasser/Methanol (1/4) wurden über Nacht am Rückfluss

gekocht. Anschliessend wurde der Alkohol am Rotationsverdampfer abgezogen, der Rückstand mit ca. 200 ml Wasser verdünnt und dann unter Eiskühlung mit ca. 70 ml conz. Salzsäure bis pH = 1 versetzt. Das Gemisch wurde mehrmals mit Äther ausgeschüttelt, die vereinigten organischen Phasen wurden mit gesättigter Natriumchlorid-Lösung gewaschen und zur Trockne eingeengt. Der Rückstand wurde 4 Stunden unter $CO_2$-Entwicklung auf 130 °C erhitzt und anschliessend im Vakuum destilliert. Es wurden 55,5 g vom $Kp_1$ = 98–107 °C erhalten.

IR (Film): 3030, 2670, 2600, 1715, 1650 (schwach)/cm.

6c) (2-Cyclohexenyl)-essigsäure-methylester

Die in der vorigen Reaktionsstufe erhaltenen 55,5 g Carbonsäure wurden mit Diazomethan umgesetzt und anschliessend im Vakuum destilliert. Man erhielt 46 g vom $Kp_{0,1}$ = 34–36 °C.

IR (Film): 1740, 1650 (schwach), 1165/cm.

6d) 3-(Cyclohexen-2-en-yl)-2-oxopropanphosphonsäure-dimethylester

Zu einer Lösung von 25,6 g Methanphosphonsäure-dimethylester in 300 ml abs. Tetrahydrofuran tropfte man unter Argon bei −60 °C 113 ml einer 1,77 m Butyllithium-Lösung in Hexan. Nach 15 Minuten wurde eine Lösung von 15,4 g des nach obiger Vorschrift erhaltenen Esters in 50 ml abs. Tetrahydrofuran zugetropft. Man hielt das Reaktionsgemisch 2 Stunden bei −65 °C und liess es dann innerhalb von 2 Stunden sich auf Raumtemperatur erwärmen. Danach wurde es mit 11,4 ml Eisessig versetzt und im Vakuum zur Trockne eingeengt. Der Rückstand wurde in einem Zweiphasensystem aus 70 ml Wasser und 330 ml Äther verteilt, die organische Phase über Magnesiumsulfat getrocknet und am Rotationsverdampfer eingeengt. Der Eindampfrückstand wurde durch Destillation bei 40 °C/0,1 mm von flüchtigen Nebenprodukten und unumgesetztem Edukt befreit und anschliessend durch Säulenchromatographie an Kieselgel mit Hexan/10–90% Essigester als Fliessmittel gereinigt. Man erhielt 13,65 g des gewünschten Phosphonats.

IR (Film): 1710, 1640 (schwach), 1260, 1025/cm.

6e) (1S,5R,6R,7R)-7-Benzoyloxy-6-[(E)-3-oxo-4-(2-cyclohexenyl)-1-butenyl]2-oxabicyclo-[3.3.0]-octan-3-on

Zu einer Suspension von 1,2 g 50%igem Natriumhydrid (Suspension in Öl) in 100 ml Dimethoxyäthan (frisch über Lithiumaluminiumhydrid destilliert) wurden unter Argon bei Raumtemperatur 6,29 g Phosphonat des Beispiels 6d) gelöst in 50 ml Dimethoxyäthan getropft. Nach Zugabe von 1,08 g trockenem Lithiumchlorid wurde die Reaktionsmischung 2 Stunden bei Raumtemperatur gerührt. Anschliessend wurde die Suspension auf −20 °C gekühlt und tropfenweise mit 7 g «Corey-Aldehyd» [J. Amer. Chem. Soc. 91, 5675 (1969)] gelöst in 160 ml Dimethoxyäthan versetzt. Danach liess man die Temperatur innerhalb von 2,5 Stunden auf 0 °C steigen. Dann versetzte man tropfenweise bei −10 °C mit 2,5 ml Eisessig, gab 150 ml Wasser zu, trennte die Phasen und extrahierte die wässrige Phase fünfmal mit Äther. Die vereinigten Phasen wurden mit 4%iger Natriumbicarbonat- und gesättigter Natriumchlorid-Lösung gewaschen, über Magnesiumsulfat getrocknet und eingeengt. Der Rückstand wurde durch Säulenchromatographie an Kieselgel mit Hexan/5–50% Essigester als Fliessmittel gereinigt. Man erhielt 8,74 g vom Schmelzpunkt 78–79 °C (Äther/Hexan).

IR (KBr): 1775, 1720, 1675, 1630, 1600, 1580, 1275, 1180, 720/cm.

6f) (1S,5R,6R,7R,3′S)-7-Benzoyloxy-6-[(E)-3-hydroxy-4-(2-cyclohexenyl)-1-butenyl]-2-oxabicyclo[3.3.0]-octan-3-on

5,27 g Natriumborhydrid wurden portionsweise unter Rühren zu einer unter Argon gehaltenen, auf −40 °C abgekühlten Lösung von 8,74 g des in der vorigen Reaktionsstufe erhaltenen Ketons (Beispiel 6e) in 280 ml abs. Methanol und 120 ml abs. Tetrahydrofuran gegeben. Nach 20 Minuten Nachrühren bei dieser Temperatur versetzte man mit 11,5 ml Eisessig und engte anschliessend am Rotationsverdampfer ein. Der Rückstand wurde mit ca. 130 ml Wasser und 280 ml Methylenchlorid aufgenommen. Die abgetrennte Wasserphase wurde mit festem Natriumchlorid versetzt und zweimal mit je ca. 200 ml Methylenchlorid extrahiert. Die vereinigten organischen Phasen wurden mit gesättigter Natriumchlorid-Lösung gewaschen, über Magnesiumsulfat getrocknet und im Vakuum eingeengt. Die Isomerentrennung des Rückstands erfolgte durch Säulenchromatographie an Kieselgel mit Hexan/10–60% Essigester als Fliessmittel. Als unpolarstes Produkt wurden 4,65 g der Titelverbindung isoliert.

IR (Film): 3470 (breit), 1770, 1715, 1600, 1580, 1275, 1175, 715/cm.

6g) (1S,5R,6R,7R)-6-[(E)-(3S)-3-Hydroxy-4-(2-cyclohexenyl)-1-butenyl]-7-hydroxy-2-oxabicyclo[3.3.0]-octan-3-on

Eine Lösung von 4,65 g des nach Beispiel 6f) erhaltenen Benzoats in 230 ml abs. Methanol wurde mit 1,6 g Kaliumcarbonat (wasserfrei) versetzt und 4 Stunden bei Raumtemperatur unter Argon gerührt. Anschliessend gab man 230 ml 0,1 n Salzsäure zur Reaktionsmischung und rührte weitere 15 Minuten. Nach Einengen der Lösung wurde mit Essigester extrahiert, die vereinigten organischen Basen wurden dann mit gesättigter Natriumchlorid-Lösung gewaschen, getrocknet und im Vakuum eingeengt. Man erhielt 3,2 g des gewünschten Diols. Eine chromatographische Reinigung war nicht erforderlich.

IR (Film): 3460 (breit), 1770, 1635, 1180/cm.

6h) (1S,5R,6R,7R)-6-[(E)-(3S)-3-(Tetrahydropyran-2-yloxy)-4-(2-cyclohexenyl)-1-butenyl]-7-(tetrahydropyran-2-yloxy)-2-oxabicyclo[3.3.0]-octan-3-on

Zu einer Lösung von 3,2 g des in der vorigen Reaktionsstufe erhaltenen Diols in 120 ml abs. Methylenchlorid gab man 2,9 ml frisch über Kaliumhydroxid destilliertes Dihydropyran und 450 mg Pyridin-p-Toluolsulfonat. Nach 19stündigem Rühren bei Raumtemperatur wurde die Reaktionslösung mit halbgesättigter Natriumchlorid-Lösung gewaschen, über Magnesiumsulfat getrocknet und im Vakuum eingeengt. Der Eindampfrückstand wurde durch Säulenchromatographie an Kieselgel mit Äther als Fliessmittel gereinigt. Es wurden 4,6 g der Titelverbindung erhalten.

IR (KBr): 1770, 1760, 1175, 1130, 1075, 1025, 975, 810/cm.

6i) (2RS,3aR,4R,5R,6aS)-4-[(E)-(3S)-3-(Tetrahydropyran-2-yloxy)-4-(3-cyclohexenyl)-1-butenyl]-5-(tetrahydropyran-2-yloxy)-perhydrocyclopenta[b]-furan-2-ol

Zu einer auf −70 °C gekühlten Lösung der in der vorigen Reaktionsstufe erhaltenen 4,6 g Lakton (Beispiel 6h) in 150 ml abs.Toluol unter Argon wurden 13,7 ml einer 20%igen Diisobutylaluminiumhydrid-Lösung in Toluol innerhalb von 10–15 Minuten getropft. Man rührte 5 Minuten nach, gab dann tropfenweise 1 ml Isopropanol und bei 0 °C 16 ml Wassser zu und liess weitere 10 Minuten rühren. Der entstandene weisse körnige Niederschlag wurde über eine Fritte abgetrennt und mit Essigester nachgewaschen. Die organischen Phasen wurden mit gesättigter Natriumchlorid-Lösung dreimal gewaschen, über Magnesiumsulfat getrocknet und im Vakuum eingeengt. Man erhielt 4,6 g Öl, die ohne weitere Reinigung in die nächste Reaktionsstufe eingesetzt wurden.

6j) (5Z,13E)-(8R,9S,11R,12R,15S)-9-Hydroxy-11,15-bis-(tetrahydropyran-2-yloxy)-16-(2-cyclohexenyl)-17,18,19,20-tetranor-5,13-prostadiensäure

96 ml einer Lösung von Methansulfinylmethylnatrium in abs. Dimethylsulfoxid (Lösung: 7,5 g 50%ige Natriumhydrid-Suspension in 150 ml abs. Dimethylsulfoxid eine halbe Stunde bei 70 °C rühren) wurden zu einer Lösung von 22 g 4-Carboxybutyltriphenylphosphoniumbromid (1,5 Stunden bei 75–80 °C an der Ölpumpe getrocknet) in 90 ml abs. Dimethylsulfoxid bei ca. 15 °C zugetropft. Nach 30 Minuten Rühren bei Raumtemperatur wurde diese Ylenlösung bei 15 °C innerhalb von 15 Minuten zu einer Lösung der in dem vorhergehenden Reaktionsschritt erhaltenen 4,6 g Laktol (Beispiel 6i) in 90 ml abs. Dimethylsulfoxid getropft. Danach wurde die Reaktionsmischung 3 Stunden bei Raumtemperatur gerührt. Anschliessend wurde mit 200 ml Eis/Wasser-Gemisch versetzt und dreimal mit Äther extrahiert. Die wässrige Phase wurde mit 10%iger Citronensäure-Lösung auf pH = 4 eingestellt und je dreimal mit einer 1/1-Mischung aus Äther/Hexan und Methylenchlorid ausgeschüttelt. Laut analytischer Dünnschichtchromatographie konnten die Äther- und Methylenchlorid-Phasen verworfen werden. Die Äther/Hexan-Phase wurde mit gesättigter Natriumchlorid-Lösung dreimal gewaschen, über Magnesiumsulfat getrocknet und am Rotationsverdampfer eingeengt. Der Rückstand wurde durch Säulenchromatographie an Kieselgel mit Hexan/50–80% Essigester als Laufmittel gereinigt. Es wurden 3,6 g der Titel-Carbonsäure erhalten.

IR (Film): 3460 (breit), 2740, 2660, 1735, 1710, 1135, 1080, 1020, 975, 810/cm.

6k) (5Z,13E)-(8R,9S,11R,12R,15S)-9-Hydroxy-11,15-bis-(tetrahydropyran-2-yloxy)-16-(2-cyclohexenyl)-17,18,19,20-tetranor-5,13-prostadiensäure-methylester

Die in der vorigen Reaktionsstufe erhaltenen 3,6 g Carbonsäure wurden in wenig Methylenchlorid gelöst und mit so viel ätherischer Diazomethanlösung versetzt, bis keine Gasentwicklung mehr auftrat und die Gelbfärbung der Reaktionslösung bestehenblieb. Nach Entfernen des überschüssigen Diazomethans sowie des Lösungsmittels im Vakuum bei Raumtemperatur wurden 3,6 g des gewünschten Methylesters als farbloses Öl erhalten.

IR (Film): 3400 (breit), 1740, 1135, 1075, 1020, 975, 810/cm.

6l) (5Z,13E)-(8R,9S,11R,12R,15S)-11,15-bis-(Tetrahydropyran-2-yloxy)-9-tosyloxy-16-(2-cyclohexenyl)-17,18,19,20-tetranor-5,13-prostadiensäure-methylester

Eine Lösung von 3,6 g des nach Beispiel 6k) erhaltenen Carbonsäureesters in 6,3 ml trockenem Pyridin wurde bei 0 °C unter Argon mit 2,5 g p-Toluolsulfonylchlorid versetzt und 48 Stunden bei Raumtemperatur gerührt. Nach Zugabe von 3,5 ml Wasser wurde weitere 2 Stunden bei Raumtemperatur gerührt. Anschliessend wurde die Reaktionslösung mit 600 ml Äther verdünnt, nacheinander mit 20 ml Wasser, zweimal mit je 30 ml eiskalter 5%iger Schwefelsäure, mit 20 ml Wasser, mit 30 ml Natriumbicarbonat-Lösung und dann mit Wasser neutral gewaschen, über Magnesiumsulfat getrocknet und im Vakuum zur Trockne eingeengt. Die Reinigung des farblosen Öls erfolgte durch Säulenchromatographie an Kieselgel mit Hexan/30–50% Essigester als Laufmittel. Man erhielt 3,76 g der Titelverbindung.

IR (Film): 2940, 2860, 1740, 1600, 1490, 1370, 1175, 1030, 1020, 975/cm.

6m) (5Z,13E)-(8R,9R,11R,12R,15S)-9-Hydroxy-11,15-bis-(tetrahydropyran-2-yloxy)-16-(2-cyclohexenyl)-17,18,19,20-tetranor-5,13-prostadiensäure-methylester

Die in der vorigen Reaktionsstufe nach Beispiel 6l) erhaltenen 3,76 g wurden in 80 ml Dimethylsulfoxid gelöst und mit 8 g Kaliumnitrit versetzt. Nach 5stündigem Rühren bei 65 °C (hierbei färbte sich die Lösung erst grün, dann braun) wurde das Reaktionsgemisch in 300 ml gesättigte Natriumchlorid-Lösung gegossen. Man extrahierte dann fünfmal mit je 250 ml Äther, wusch die vereinigten organischen Phasen mit Wasser neutral, trocknete über Magnesiumsulfat und engte im Vakuum zur Trockne ein. Der Rückstand wurde durch Säulenchromatographie an Kieselgel mit Essigester/Hexan = 1/1 als Fliessmittel gereinigt. Man erhielt 1,42 g der gewünschten Verbindung als farbloses Öl.

IR: 3400, 2950, 2870, 1740, 1030, 1020, 975/cm.

6n) (5Z,13E)-(8R,9R,11R,12R,15S)-11,15-bis-(Tetrahydropyran-2-yloxy)-9-(p-toluolsulfonyloxy)-16-(2-cyclohexenyl)-17,18,19,20-tetranor-5,13-prostadiensäure-methylester

Zu einer Lösung von 1,4 g des in der vorigen Reaktionsstufe hergestellten 9βEg-Alkohols in 1,7 ml trockenem Pyridin wurden bei 0 °C 675 mg p-Toluolsulfonylchlorid gegeben. Danach wurde die Reaktionslösung 20 Stunden bei Raumtemperatur unter Argon gerührt. Nach Verdünnen mit

Äther wurde die Reaktionsmischung dann nacheinander mit Wasser, eiskalter 5%iger Schwefelsäure, Wasser, Natriumbicarbonat-Lösung und wieder Wasser gewaschen. Man trocknete über Magnesiumsulfat, dampfte im Vakuum ein und reinigte den Rückstand durch Säulenchromatographie an Kieselgel mit Hexan/30% Essigester als Fliessmittel. Es wurden 1,27 g der Titelverbindung erhalten.

IR: 2960, 2870, 1740, 1600, 1490, 1370, 1170, 1030, 1020, 975/cm.

6o) (5Z,13E)-(8R,9R,11R,12R,15S)-11,15-Dihydroxy-9-(p-toluolsulfonyloxy)-16-(2-cyclohexenyl)-17,18,19,20-tetranor-5,13-prostadiensäure-methylester

Die nach Beispiel 6n) erhaltenen 1,25 g 9β-Tosylat wurden 20 Stunden mit 37 ml einer Mischung aus Essigsäure/Wasser/Tetrahydrofuran (65/35/10) unter Argon bei Raumtemperatur gerührt. Anschliessend wurde das Reaktionsgemisch mit 150 ml gesättigter Natriumchlorid-Lösung versetzt, dreimal mit je 100 ml Essigester extrahiert, die organische Phase mit gesättigter Natriumbicarbonat-Lösung und danach mit Wasser neutral gewaschen, über Natriumsulfat getrocknet und im Vakuum eingeengt. Der Eindampfrückstand wurde einer Säulenchromatographie an Kieselgel mit Essigester/Hexan (2/1) als Fliessmittel unterworfen. Man erhielt 746 mg des Tosylats als farbloses Öl.

IR (Film): 3400 (breit), 2950, 2920, 2860, 1735, 1595, 1490, 1360, 1175, 1095, 975/cm.

6p) (5Z,13E)-(8R,9S,11R,12R,15S)-9-Azido-11,15-dihydroxy-16-(2-cyclohexenyl)-17,18,19,20-tetranor-5,13-prostadiensäure-methylester

Eine Lösung von 720 mg des nach Beispiel 6o) hergestellten Diols in 16 ml Hexamethylphosphorsäuretriamid wurde mit 155 mg Natriumazid versetzt und 4,5 Stunden bei 40 °C gerührt. Das abgekühlte Reaktionsgemisch wurde mit 100 ml Eiswasser versetzt, fünfmal mit je 50 ml Äther extrahiert, die organische Phase dreimal mit Natriumchlorid-Lösung gewaschen, über Magnesiumsulfat getrocknet und im Vakuum eingeengt. Die so als dünnschichtchromatographisch einheitliches Öl erhaltene Titelverbindung wurde ohne weitere Reinigung in die nächste Reaktion eingesetzt.

IR: 3400 (breit), 2960, 2930, 2870, 2100, 1735, 975/cm.

6q) (5Z,13E)-(8R,9S,11R,12R,15S)-9-Azido-11,15-dihydroxy-16-(2-cyclohexenyl)-17,18,19,20-tetranor-5,13-prostadiensäure

Das in der vorigen Reaktionsstufe erhaltene Azid (Beispiel 6p) wurde in 13,2 ml einer Lösung aus 3,6 g Kaliumhydroxid, 24 ml Wasser und 120 ml Methanol gegeben und 4 Stunden unter Argon bei Raumtemperatur gerührt. Die Reaktionslösung wurde anschliessend in 40 ml Wasser gegossen und einmal mit Äther/Hexan (1/1) gewaschen, die wässrige Phase wurde dann auf 5 °C abgekühlt, mit 10%iger Citronensäure-Lösung auf pH = 6 angesäuert und fünfmal mit je 50 ml Methylenchlorid extrahiert. Die vereinigten organischen Phasen wurden mit 100 ml gesättigter Natriumchlorid-Lösung gewaschen, über Magnesiumsulfat getrocknet und im Vakuum eingeengt. Der Eindampfrückstand wurde einer Reinigung durch Säulenchromatographie an Kieselgel mit Essigester/0–5% Methanol als Laufmittel unterworfen. Man erhielt 255 mg der Titelverbindung als farbloses Öl.

IR (Film): 3400 (breit), 2950, 2920, 2875, 2120, 1705, 975/cm.

Beispiel 7

(13E)-(11R,15S,16RS)-11,15-Dihydroxy-16-methyl-16-(2-cyclopenten-1-yl)-17,18,19,20-tetranor-9α,6-nitrilo-13-prostensäure

Eine Lösung von 260 mg (5Z,13E)-(8R,9S,11R,12R,15S,16RS)-9-Azido-11,15-dihydroxy-16-methyl-16-(2-cyclopenten-1-yl)-17,18,19,20-tetranor-5,13-prostadiensäure in 25 ml Essigsäureäthylester wurde 27 Stunden bei 80 °C unter Argon gerührt. Anschliessend wurde im Vakuum eingeengt und der Rückstand durch Säulenchromatographie an Kieselgel mit Essigester/5–50% Methanol als Fliessmittel gereinigt. Man erhielt 120 mg der Titelverbindung als farbloses Öl.

IR (Film): 3420 (breit), 2960, 2920, 2860, 1710, 1640, 1080, 970/cm.

Das Ausgangsmaterial für die obige Titelverbindung wurde wie folgt hergestellt:

7a) 2-(2-Cyclopenten-1-yl)-2-methylmalonsäure-diäthylester

52,4 g in kleine Stücke geschnittenes Natrium wurden in einem mit Tropftrichter, Rückflusskühler und KPG-Rührer versehenen Dreihalskolben gegeben. Unter einer Argon-Atmosphäre wurden innerhalb von 3 Stunden 790 ml abs. Äthanol so dazu getropft, dass die Reaktionsmischung lebhaft siedete. Zu der heissen, leicht trüben Alkoholat-Lösung wurden innerhalb von 1,5 Stunden 157 g Methylmalonsäurediäthylester zugetropft. Man liess eine Stunde nachrühren und gab dann zu der abgekühlten Lösung innerhalb einer Stunde tropfenweise 205 g trans-1,2-Dibromcyclopentan. Danach wurde 17 Stunden am Rückfluss gekocht. Nach Einengen der Reaktionslösung am Rotationsverdampfer wurde der Rückstand mit Äther und verdünnter Salzsäure versetzt, die organische Phase dann mit gesättigter Natriumchlorid-Lösung neutral gewaschen, über Natriumsulfat getrocknet und im Vakuum destilliert. Man erhielt 170 g vom $Kp_{1,5}$ = 90–105 °C.

IR (Film): 1731, 1250, 1100/cm.

7b) 2-(2-Cyclopenten-1-yl)-2-methyl-malonsäure

156 g des in der vorigen Reaktionsstufe erhaltenen Diesters wurden zusammen mit 83 g Natriumhydroxid in 680 ml Wasser 5,5 Stunden unter Rückfluss erhitzt. Danach wurde am Rotationsverdampfer eingeengt und unter Eiskühlung tropfenweise mit konz. Salzsäure bis zum pH-Wert 1 versetzt. Der Niederschlag wurde gesammelt, mit Wasser gewaschen und in Äther gelöst. Die Ätherphase wurde mit gesättigter Natriumchlorid-Lösung gewaschen, über Magnesiumsulfat getrocknet und im Vakuum eingeengt. Der Rückstand wurde aus Äther/Toluol kristallisiert. Es wurden 112 g vom Fp. 153 °C erhalten.

IR (KBr): 2630, 2530, 1715, 1275/cm.

7c) 2-(2-Cyclopenten-1-yl)-propionsäure

Die in der vorigen Reaktionsstufe erhaltenen 112 g wurden eine Stunde unter Kohlendioxid-Entwicklung auf 176 °C erhitzt. Anschliessend wurde das Produkt im Vakuum destilliert. Man erhielt 84,4 g der Titelverbindung mit $Kp_{0,7}$ = 81–83 °C.

IR (KBr): 2650, 2520, 1715, 1270/cm.

7d) 2-(2-Cyclopenten-1-yl)-propionsäure-methylester

Die nach obiger Vorschrift erhaltenen 84 g Carbonsäure wurden mit so viel ätherischer Diazomethan-Lösung versetzt, bis sich kein Stickstoff mehr bei Zugabe des Reagenzes entwickelte und die Gelbfärbung der Lösung bestehenblieb. Das Lösungsmittel wurde dann zusammen mit überschüssigem Diazomethan im Vakuum bei Raumtemperatur entfernt und der Rückstand fraktioniert. Es wurden 87 g des Esters mit $Kp_{0,8}$ = 43–44 °C erhalten.

IR (Film): 1740, 1620 (schwach), 1160/cm.

7e) [3-(2-Cyclopenten-1-yl)-2-oxo-butan]-phosphonsäure-dimethylester

382 ml einer 1,31 m Butyllithium-Lösung in Hexan wurden unter Argon bei −60 °C zu einer Lösung von 62 g Methanphosphonsäuredimethylester in 800 ml abs. Tetrahydrofuran getropft. Nach einer halben Stunde wurde bei der gleichen Temperatur eine Lösung von 30,84 g des in der vorigen Reaktionsstufe erhaltenen Carbonsäureesters in 75 ml abs. Tetrahydrofuran zugetropft. Man hielt das Reaktionsgemisch 3 Stunden auf −60 °C und liess es sich dann auf Raumtemperatur erwärmen. Danach wurde es mit 28,6 ml Eisessig versetzt und am Rotationsverdampfer eingeengt. Der Rückstand wurde in einem Zweiphasensystem aus Äther/Wasser verteilt und die wässrige Phase mehrmals mit Äther extrahiert. Die vereinigten organischen Phasen wurden mit gesättigter Natriumchlorid-Lösung gewaschen, über Natriumsulfat getrocknet und im Vakuum eingeengt. Der Rückstand wurde einer Säulenchromatographie an Kieselgel mit Hexan/50–100% Essigester sowie Essigester/0–7% Methanol als Fliessmittel unterworfen. Man erhielt 35,6 g des gewünschten Phosphonats.

IR (Film): 1710, 1258, 1033/cm.

7f) (1S,5R,6R,7R)-7-Benzoyloxy-6-[(E)-(4RS)-4-methyl-3-oxo-4-(2-cyclopenten-1-yl)-1-butenyl]-2-oxabicyclo[3.3.0]octan-3-on

Zu einer Suspension von 1,56 g 50%igem (in Öl suspendiertem) Natriumhydrid in 170 ml frisch über Lithiumaluminiumhydrid destilliertem Dimethoxyäthan wurden unter Argon bei Raumtemperatur 8,0 g des in der vorigen Reaktionsstufe erhaltenen Phosphonats (gelöst in 85 ml abs. Dimethoxyäthan) getropft. Nach Zugabe von 1,38 g Lithiumchlorid (vorher im Vakuum 2 Stunden bei 50 °C getrocknet) wurde das Reaktionsgemisch 2 Stunden bei Raumtemperatur gerührt. Anschliessend wurde die Suspension auf −20 °C abgekühlt und tropfenweise mit einer Lösung von 8,9 g (1S,5R,6R,7R)-6-Formyl-7-benzoyloxy-2-oxabicyclo[3.3.0]octan-3-on [E.J. Corey et al., J. Amer. Chem. Soc. 91, 5675 (1969)] in 255 ml abs. Dimethoxyäthan innerhalb einer halben Stunde versetzt. Danach liess man die Temperatur innerhalb von 4,5 Stunden unter Rühren auf −5 °C ansteigen. Nach erfolgter analytischer Dünnschichtchromatographie-Kontrolle wurden anschliessend bei −10 °C 3,3 ml Eisessig tropfenweise zugegeben. Danach versetzte man mit 450 ml Wasser, trennte die Phasen, schüttelte die wässrige dreimal mit je ca. 200 ml Äther aus, vereinigte die organischen Phasen und wusch sie mit 4%iger Natriumbicarbonat- und gesättigter Natriumchloridlösung. Nach Trocknen über Magnesiumsulfat wurde das Lösungsmittel am Rotationsverdampfer entfernt. Nach Reinigung des Rückstands durch Säulenchromatographie an Kieselgel mit Hexan/50–100% Essigester als Fliessmittel erhielt man 11,85 g der Titelverbindung vom Fp. 85–87,5 °C.

IR (KBr): 1766, 1713, 1688, 1622, 1602, 1582, 1274, 1160, 711/cm.

7g) (1S,5R,6R,7R)-7-Benzoyloxy-6-[(E)-(3S,4RS)-3-hydroxy-4-methyl-4-(2-cyclopenten-1-yl)-1-butenyl]-2-oxabicyclo[3.3.0]octan-3-on

Zu einer auf −40 °C gekühlten Lösung von 11,85 g des in der vorigen Reaktionsstufe erhaltenen Ketons in 350 ml abs. Methanol wurden portionsweise 7,18 g Natriumborhydrid gegeben. Nach 75 Minuten Nachrühren bei dieser Temperatur wurden – ebenfalls bei −40 °C – 15,4 ml Eisessig zur Reaktionslösung getropft. Nach Entfernen des Lösungsmittels am Rotationsverdampfer wurde der Rückstand mit einem Zweiphasengemisch aus 200 ml Wasser und 300 ml Methylenchlorid versetzt, die abgetrennte Wasserphase mit festem Natriumchlorid versetzt und zweimal mit je ca. 200 ml Methylenchlorid extrahiert. Die vereinigten organischen Phasen wurden mit gesättigter Natriumchlorid-Lösung gewaschen, über Magnesiumsulfat getrocknet und im Vakuum eingeengt. Die Isomerentrennung des Rückstands erfolgte durch Säulenchromatographie an Kieselgel mit Hexan/50–100% Essigester als Fliessmittel. Als unpolarstes Produkt wurden 5,7 g der Titelverbindung isoliert.

IR (Film): 3490, 1770, 1718, 1602, 1584, 1275, 1178, 713/cm.

7h) (1S,5R,6R,7R)-6-[(E)-(3S,4RS)-3-Hydroxy-4-methyl-4-(2-cyclopenten-1-yl)-1-butenyl]-7-hydroxy-2-oxabicyclo[3.3.0]-octan-3-on

Eine Lösung von 8 g des in der vorigen Reaktionsstufe erhaltenen Benzoats in 396 ml abs. Methanol wurde mit 2,8 g wasserfreiem Kaliumcarbonat versetzt und 3 Stunden bei Raumtemperatur und unter Argon gerührt. Danach gab man 396 ml 0,1 n Salzsäure zur Reaktionslösung und rührte weitere 15 Minuten. Die Lösung wurde eingeengt und mit Essigester extrahiert. Die vereinigten organischen Phasen wurden mit gesättigter Natriumchlorid-Lösung gewaschen, getrocknet und im Vakuum eingeengt. Der Rückstand wurde durch Säulenchromatographie an Kieselgel mit Hexan/80–100% Essigester als Fliessmittel gereinigt. Man erhielt 4,63 g der Titelverbindung.

IR (Film): 3420 (breit), 1762, 1670 (schwach), 1174/cm.

7i) (1S,5R,6R,7R)-6-[(E)-(3S,4RS)-3-Tetrahydropyran-2-yloxy)-4-methyl-4-(2-cyclopenten-1-yl)-1-butenyl]-7-(tetrahydropyran-2-yloxy)-2-oxabicyclo[3.3.0]octan-3-on

Zu einer Lösung von 3,54 g des in der vorigen Reaktionsstufe erhaltenen Diols in 190 ml abs. Methylenchlorid gab man bei 0 °C 1,64 ml frisch über Kaliumhydroxid destilliertes Dihydropyran und 33 mg p-Toluolsulfonsäure. Nach 3stündigem Rühren wurde die Reaktionslösung mit gesättigter Natriumbicarbonat-Lösung und Wasser gewaschen, über Natriumsulfat getrocknet und im Vakuum eingeengt. Der Rückstand wurde durch Säulenchromatographie an Kieselgel mit Hexan/50–100% Essigester als Fliessmittel gereinigt. Es wurden 5,3 g des Di-THP-Äthers isoliert.

IR (Film): 1770, 1175, 1130, 1075, 1020, 970, 810/cm.

7j) (2RS,3aR,4R,5R,6aS)-4-[(E)-(3S)-(4RS)-3-(Tetrahydropyran-2-yloxy)-4-methyl-4-(2-cyclopenten-1-yl)-1-butenyl]-5-(tetrahydropyran-2-yloxy)-perhydrocyclopenta[b]-furan-2-ol

Zu einer auf −70 °C gekühlten Lösung der in der vorigen Reaktionsstufe erhaltenen 5,3 g Lakton (Beispiel 7i) in 150 ml abs. Toluol unter Argon wurden 15,7 ml einer 20%igen Diisobutylaluminiumhydrid-Lösung in Toluol innerhalb von 10–15 Minuten getropft. Man rührte 5 Minuten nach, gab dann tropfenweise 1 ml Isopropanol und bei 0 °C 16 ml Wasser zu und liess weitere 10 Minuten rühren. Der entstandene weisse körnige Niederschlag wurde über eine Fritte abgetrennt und mit Essigester nachgewaschen. Die organischen Phasen wurden mit gesättigter Natriumchlorid-Lösung dreimal gewaschen, über Magnesiumsulfat getrocknet und im Vakuum eingeengt. Man erhielt 5,3 g Öl, die ohne weitere Reinigung in die nächste Reaktionsstufe eingesetzt wurden.

7k) (5Z,13E)-(8R,9S,11R,12R,15S,16RS)-9-Hydroxy-11,15-bis-(tetrahydropyran-2-yloxy)-16-methyl-16-(2-cyclopenten-1-yl)-17,18,19,20-tetranor-5,13-prostadiensäure

111 ml einer Lösung von Methansulfinylmethylnatrium in abs. Dimethylsulfoxid (Lösung: 7,5 g 50%ige Natriumhydrid-Suspension in 150 ml abs. Dimethylsulfoxid eine halbe Stunde bei 70 °C rühren) wurden zu einer Lösung von 25 g 4-Carboxybutyltriphenylphosphoniumbromid (1,5 Stunden bei 75–80 °C an der Ölpumpe getrocknet) in 90 ml abs. Dimethylsulfoxid bei ca. 15 °C zugetropft. Nach 30 Minuten Rühren bei Raumtemperatur wurde diese Ylenlösung bei 15 °C innerhalb von 15 Minuten zu einer Lösung der in dem vorhergehenden Reaktionsschritt erhaltenen 5,3 g Laktol (Beispiel 7j) in 90 ml abs. Dimethylsulfoxid getropft. Danach wurde die Reaktionsmischung 3 Stunden bei Raumtemperatur gerührt. Anschliessend wurde mit 200 ml Eis/Wasser-Gemisch versetzt und dreimal mit Äther extrahiert. Die wässrige Phase wurde mit 10%iger Citronensäure-Lösung auf pH = 4 eingestellt und je dreimal mit einer 1/1-Mischung aus Äther/Hexan und Methylenchlorid ausgeschüttelt. Laut analytischer Dünnschichtchromatographie konnten die Äther- und Methylenchlorid-Phasen verworfen werden. Die Äther/Hexan-Phase wurde mit gesättigter Natriumchlorid-Lösung dreimal gewaschen, über Magnesiumsulfat getrocknet und am Rotationsverdampfer eingeengt. Der Rückstand wurde durch Säulenchromatographie an Kieselgel mit Hexan/50–80% Essigester als Laufmittel gereinigt. Es wurden 4,3 g der Titel-Carbonsäure erhalten.

IR (Film): 3460 (breit), 2740, 2660, 1730, 1710, 1120, 1077, 1025, 975/cm.

7l) (5Z,13E)-(8R,9S,11R,12R,15S,16RS)-9-Hydroxy-11,15-bis-(tetrahydropyran-2-yloxy)-16-methyl-16-(2-cyclopenten-1-yl)-17,18,19,20-tetranor-5,13-prostadiensäure-methylester

Die in der vorigen Reaktionsstufe erhaltenen 4,3 g Carbonsäure wurden in wenig Methylenchlorid gelöst und mit so viel ätherischer Diazomethanlösung versetzt, bis keine Gasentwicklung mehr auftrat und die Gelbfärbung der Reaktionslösung bestehenblieb. Nach Entfernen des überschüssigen Diazomethans sowie des Lösungsmittels im Vakuum bei Raumtemperatur wurden 4,3 g des gewünschten Methylesters als farbloses Öl erhalten.

IR (Film): 3470 (breit), 1740, 1130, 1080, 1020, 975/cm.

7m) (5Z,13E)-(8R,9S,11R,12R,15S,16RS)-11,15-bis-(tetrahydropyran-2-yloxy)-9-tosyloxy-16-methyl-16-(2-cyclopenten-1-yl)-17,18,19,20-tetranor-5,13-prostadiensäure-methylester

Eine Lösung von 4,3 g des nach Beispiel 7l) erhaltenen Carbonsäureesters in 7,5 ml trockenem Pyridin wurde bei 0 °C unter Argon mit 3 g p-Toluolsulfonylchlorid versetzt und 48 Stunden bei Raumtemperatur gerührt. Nach Zugabe von 4 ml Wasser wurde weitere 2 Stunden bei Raumtemperatur gerührt. Anschliessend wurde die Reaktionslösung mit 600 ml Äther verdünnt, nacheinander mit 20 ml Wasser, zweimal mit je 30 ml eiskalter 5%iger Schwefelsäure, mit 20 ml Wasser, mit 30 ml Natriumbicarbonat-Lösung und dann mit Wasser neutral gewaschen, über Magnesiumsulfat getrocknet und im Vakuum zur Trockne eingeengt. Die Reinigung des farblosen Öls erfolgte durch Säulenchromatographie an Kieselgel mit Hexan/30–50% Essigester als Laufmittel. Man erhielt 4,4 g der Titelverbindung.

IR (Film): 1740, 1600, 1495, 1370, 1180, 1130, 1080, 1020, 975/cm.

7n) (5Z,13E)-(8R,9R,11R,12R,15S,16RS)-9-Hydroxy-11,15-bis-(tetrahydropyran-2-yloxy)-16-methyl-16-(2-cyclopenten-1-yl)-17,18,19,20-tetranor-5,13-prostadiensäure-methylester

Die in der vorigen Reaktionsstufe nach Beispiel 7m) erhaltenen 4,4 g wurden in 80 ml Dimethylsulfoxid gelöst und mit 9,5 g Kaliumnitrit versetzt. Nach 6stündigem Rühren bei 65 °C (hierbei färbte sich die Lösung erst grün, dann braun) wurde das Reaktionsgemisch in 300 ml gesättigte Natriumchlorid-Lösung gegossen. Man extrahierte dann fünfmal mit je 250 ml Äther, wusch die vereinigten organischen Phasen mit Wasser neutral, trocknete über Magnesiumsulfat und engte im Vakuum zur Trockne ein. Der Rückstand wurde durch Säulenchromatographie an Kieselgel mit Essigester/Hexan = 1/1 als Fliessmittel gereinigt. Man erhielt 1,7 g der gewünschten Verbindung als farbloses Öl.

IR: 3400, 2950, 2860, 1740, 1130, 1080, 1020, 970/cm.

7o) (5Z,13E)-(8R,9R,11R,12R,15S,16RS)-11,15-bis-(tetrahydropyran-2-yloxy)-9-(p-toluolsulfonyloxy)-16-methyl-16-(2-cyclopenten-1-yl)-17,18,19,20-tetranor-5,13-prostadiensäure-methylester

Zu einer Lösung von 1,7 g des in der vorigen Reaktionsstufe hergestellten 9β-Alkohols in 2 ml trockenem Pyridin wurden bei 0 °C 820 mg p-Toluolsulfonylchlorid gegeben. Danach wurde die Reaktionslösung 20 Stunden bei Raumtemperatur unter Argon gerührt. Nach Verdünnen mit Äther wurde die Reaktionsmischung dann nacheinander mit Wasser, eiskalter 5%iger Schwefelsäure, Wasser, Natriumbicarbonat-Lösung und wieder Wasser gewaschen. Man trocknete über Magnesiumsulfat, dampfte im Vakuum ein und reinigte den Rückstand durch Säulenchromatographie an Kieselgel mit Hexan/30% Essigester als Fliessmittel. Es wurden 1,54 g der Titelverbindung erhalten.

IR: 2960, 2870, 1735, 1600, 1490, 1370, 1175, 1130, 1020, 975/cm.

7p) (5Z,13E)-(8R,9R,11R,12R,15S,16RS)-11,15-Dihydroxy-9-(p-toluolsulfonyloxy)-16-methyl-16-(2-cyclo-penten-1-yl)-17,18,19,20-tetranor-5,13-prostadiensäure-methylester

Die nach Beispiel 7o) erhaltenen 1,5 g 9β-Tosylat wurden 20 Stunden mit 45 ml einer Mischung aus Essigsäure/Wasser/Tetrahydrofuran (65/35/10) unter Argon bei Raumtemperatur gerührt. Anschliessend wurde das Reaktionsgemisch mit 150 ml gesättigter Natriumchlorid-Lösung versetzt, dreimal mit je 100 ml Essigester extrahiert, die organische Phase mit gesättigter Natriumbicarbonat-Lösung und danach mit Wasser neutral gewaschen, über Natriumsulfat getrocknet und im Vakuum eingeengt. Der Eindampfrückstand wurde einer Säulenchromatographie an Kieselgel mit Essigester/Hexan (2/1) als Fliessmittel unterworfen. Man erhielt 900 mg des Tosylats als farbloses Öl.

IR (Film): 3400 (breit), 2950, 2920, 2860, 1735, 1595, 1490, 1360, 1175, 1090, 975/cm.

7q) (5Z,13E)-(8R,9S,11R,12R,15S,16RS)-9-Azido-11,15-dihydroxy-16-methyl-16-(2-cyclopenten-1-yl)-17,18,19,20-tetranor-5,13-prostadiensäure-methylester

Eine Lösung von 850 mg des nach Beispiel 7p) hergestellten Diols in 19 ml Hexamethylphosphorsäuretriamid wurde mit 185 mg Natriumazid versetzt und 4 Stunden bei 40 °C gerührt. Das abgekühlte Reaktionsgemisch wurde mit 100 ml Eiswasser versetzt, fünfmal mit je 50 ml Äther extrahiert, die organische Phase dreimal mit Natriumchlorid-Lösung gewaschen, über Magnesiumsulfat getrocknet und im Vakuum eingeengt. Die so als dünnschichtchromatographisch einheitliches Öl erhaltene Titelverbindung wurde ohne weitere Reinigung in die nächste Reaktion eingesetzt.

IR: 3400 (breit), 2960, 2930, 2870, 2100, 1735, 970/cm.

7r) (5Z,13E)-(8R,9S,11R,12R,15S,16RS)-9-Azido-11,15-dihydroxy-16-methyl-16-(2-cyclopenten-1-yl)-17,18,19,20-tetranor-5,13-prostadiensäure

Das in der vorigen Reaktionsstufe erhaltene Azid (Beispiel 7q) wurde in 16 ml einer Lösung aus 3,6 g Kaliumhydroxid, 24 ml Wasser und 120 ml Methanol gegeben und 4 Stunden unter Argon bei Raumtemperatur gerührt. Die Reaktionslösung wurde anschliessend in 40 ml Wasser gegossen und einmal mit Äther/Hexan (1/1) gewaschen, die wässrige Phase wurde dann auf 5 °C abgekühlt, mit 10%iger Citronensäure-Lösung auf pH = 6 angesäuert und fünfmal mit je 50 ml Methylenchlorid extrahiert. Die vereinigten organischen Phasen wurden mit 100 ml gesättigter Natriumchlorid-Lösung gewaschen, über Magnesiumsulfat getrocknet und im Vakuum eingeengt. Der Eindampfrückstand wurde einer Reinigung durch Säulenchromatographie an Kieselgel mit Essigester/0–5% Methanol als Laufmittel unterworfen. Man erhielt 290 mg der Titelverbindung als farbloses Öl.

IR (Film): 3420 (breit), 2950, 2920, 2875, 2120, 1705, 970/cm.

Beispiel 8

(13E)-(11R,15S,16RS)-11,15-Dihydroxy-16,19-dimethyl-9α,6-nitrilo-13,18-prostadiensäure-methylester

Eine Lösung von 100 mg (13E)-(11R,15S,16RS)-11,15-Dihydroxy-16,19-dimethyl-9α,6-nitrilo-13,18-prostadiensäure (Beispiel 1) in wenig Methylenchlorid wurde bei −10 °C mit so viel ätherischer Diazomethanlösung versetzt, bis keine Gasentwicklung mehr auftrat und die Gelbfärbung der Lösung bestehenblieb. Nach Entfernen des überschüssigen Diazomethans im Vakuum bei Raumtemperatur wurden 80 mg der Titelverbindung als farbloses Öl erhalten.

IR (Film): 3450 (breit), 2960, 2860, 1735, 975/cm.

Beispiel 9

Tris-(hydroxymethyl)-aminomethansalz von (13E)-(11R,15S,16RS)-11,15-Dihydroxy-16,19-dimethyl-9α,6-nitrilo-13,18-prostadiensäure

Zu einer Lösung von 100 mg (13E)-(11R,15S,16RS)-11,15-Dihydroxy-16,19-dimethyl-9α,6-nitrilo-13,18-prostadiensäure (Beispiel 1) in 20 ml Azetonitril gab man bei 65 °C eine Lösung von 34 mg Tris(hydroxymethyl)-aminomethan in 0,2 ml Wasser. Unter Rühren liess man abkühlen, dekantierte nach 14 Stunden und trocknete den Rückstand bei 25 °C/0,1 Torr. Es wurden 85 mg der Titelverbindung erhalten.

Beispiel 10

(13E)-(11R,15S,16RS)-11,15-Dihydroxy-16,19-dimethyl-9α,6-nitrilo-13,18-prostadiensäure-butylester

In Analogie zu Beispiel 8 erhielt man aus der nach Beispiel 1 dargestellten Säure mit Diazobutan die Titelverbindung als farbloses Öl.

IR (Film): 3430 (breit), 2960, 2920, 2860, 1740, 970/cm.

Beispiel 11

(13E)-(11R,15S,16RS)-11,15-Dihydroxy-16,20-dimethyl-9α,6-nitrilo-13-prosten-19-in-säure

Eine Lösung von 240 mg

(5Z,13E)-(9S,11R,15S,16RS)-9-Azido-11,15-dihydroxy-16,20-dimethyl-5,13-prostadien-19-in-säure

in 20 ml Essigsäureäthylester erhitzte man 24 Stunden unter Argon auf 70–75 °C. Nach Verdampfen des Lösungsmittels im Vakuum wurde der Rückstand an Kieselgel mit Methylenchlorid/10–30% Isopropanol als Fliessmittel gereinigt. Man erhielt 160 mg der Titelverbindung als farbloses Öl.

IR (Film): 3600, 3400 (breit), 2960, 2860, 1715, 1640, 1020, 975/cm.

Das Ausgangsmaterial für die obige Titelverbindung wurde wie folgt hergestellt:

11a) (1S,5R,6R,7R)-7-Benzoyloxy-6-[(E)-(4RS)-4-methyl-3-oxo-non-1-en-7-inyl]-2-oxabicyclo-[3.3.0]-octan-3-on

In Analogie zu Beispiel 1g) wurden aus 5,4 g

3-Methyl-2-oxo-oct-6-inyl-phosphonsäure-dimethylester

und 6 g

(1S,5R,6R,7R)-6-Formyl-7-benzoyloxy-2-oxa-bicyclo-[3.3.0]-octan-3-on

[E.J. Corey et al., J. Amer. Chem. Soc. 91, 5675 (1969)] 7,4 g der Titelverbindung synthetisiert.

IR (Film): 1775, 1720, 1686, 1670, 1620, 1600, 1580, 1270, 1182, 715/cm.

11b) (1S,5R,6R,7R)-7-Benzoyloxy-6-[(E)-(3S,4RS)-3-hydroxy-4-methyl-non-1-en-7-inyl]-2-oxabicyclo-[3.3.0]-octan-3-on

Analog der Vorschrift in Beispiel 1h) wurden die in der vorigen Reaktionsstufe erhaltenen 7,4 g Keton mit Natriumborhydrid umgesetzt. Man isolierte 4 g der Titelverbindung als unpolarstes Produkt bei der Säulenchromatographie.

IR (Film): 3480 (breit), 1775, 1715, 1603, 1590, 1275, 1180, 715/cm.

11c) (1S,5R,6R,7R)-6-[(E)-(3S,4RS)-3-Hydroxy-4-methyl-non-1-en-7-inyl]-7-hydroxy-2-oxa-bicyclo-[3.3.0]-octan-3-on

In Analogie zu Beispiel 1i) wurden die in der vorigen Reaktionsstufe erhaltenen 4 g umgeestert. Man erhielt 2,6 g der Titelverbindung als farbloses Öl.

IR (Film): 3460 (breit), 1765, 1175, 1030, 970/cm.

11d) (1S,5R,6R,7R)-6-[(E)-(3S,4RS)-3-(Tetrahydropyran-2-yloxy)-4-methyl-non-1-en-7-inyl]-7-(tetrahydropyran-2-yloxy)-2-oxabicyclo-[3.3.0]-octan-3-on

Analog zur Vorschrift in Beispiel 1j) wurde die nach Beispiel 11c) erhaltenen 2,6 g zu 3,8 g Di-THP-Äther umgesetzt.

IR (Film): 1770, 1180, 1130, 1075, 1020, 975, 810/cm.

11e) (2RS,3aR,4R,5R,6aS)-4-[(E)-(3S,4RS)-3-(tetrahydropyran-2-yloxy)-4-methyl-non-1-en-7-inyl]-5-(tetrahydropyran-2-yloxy)-perhydro-cyclopenta[b]-furan-2-ol

In Analogie zu Beispiel 1h) wurden 3,8 g Lakton (Beispiel 11d) mit Diisobutylaluminiumhydrid zu 4 g Lactol umgesetzt, die ohne weitere Reinigung in die nächste Stufe eingesetzt wurden.

11f) (5Z,13E)-(8R,9S,11R,12R,15S,16RS)-9-Hydroxy-11,15-bis-(tetrahydropyran-2-yloxy)-16,20-dimethyl-5,13-prostadien-19-in-säure

Die nach Beispiel 11e) erhaltenen 4 g Laktol wurden analog der Vorschrift in Beispiel 1l) in einer Wittig-Reaktion zu 2,9 g Säure umgesetzt.

IR (Film): 3480 (breit), 2730, 2660, 1735, 1710, 1130, 1080, 1020, 975, 810/cm.

11g) (5Z,13E)-(8R,9S,11R,12R,15S,16RS)-9-Hydroxy-11,15-bis-(tetrahydropyran-2-yloxy)-16,20-dimethyl-5,13-prostadien-19-in-säuremethylester

Analog der Vorschrift in Beispiel 1m) wurden 2,9 g Carbonsäure (Beispiel 1f) mit Diazomethan zu 2,8 g Ester umgesetzt.

IR (Film): 3400 (breit), 1738, 1132, 1078, 1020, 975, 810/cm.

11h) (5Z,13E)-(8R,9S,11R,12R,15S,16RS)-16,20-Dimethyl-11,15-bis-(tetrahydropyran-2-yloxy)-9-tosyloxy-5,13-prostadien-19-in-säuremethylester

2,8 g des nach Beispiel 11g) erhaltenen Esters wurden in Analogie zur Reaktion nach Beispiel 1n) in 2,7 g öliges 9α-Tosylat überführt.

IR (Film): 2960, 2870, 1735, 1602, 1360, 1175, 975/cm.

11i) (5Z,13E)-(8R,9R,11R,12R,15S,16RS)-9-Hydroxy-11,15-bis-(tetrahydropyran-2-yloxy)-16,20-dimethyl-5,13-prostadien-19-in-säuremethylester

2,7 g des nach Beispiel 11h) hergestellten Tosylats wurden in Analogie zu Beispiel 1o) mit Kaliumnitrit umgesetzt. Man erhielt 1 g des 9β-Alkohols als farbloses Öl.

IR (Film): 3450, 2950, 1735, 980/cm.

11j) (5Z,13E)-(8R,9R,11R,12R,15S,16RS)-16,20-Dimethyl-11,15-bis-(tetrahydropyran-2-yloxy)-9-(p-toluolsulfonyloxy)-5,13-prostadien-19-in-säuremethylester

In Analogie zu Beispiel 1p) erhielt man aus 1 g des nach Beispiel 11i) hergestellten 9β-Alkohols 1,1 g des 9β-Tosylats als Öl.

IR (Film): 2960, 2860, 1735, 1600, 1495, 1365, 1175, 1030, 1020, 975/cm.

11k) (5Z,13E)-(8R,9R,11R,12R,15S,16RS)-11,15-Dihydroxy-16,20-dimethyl-9-(p-toluolsulfonyloxy)-5,13-prostadien-19-in-säuremethylester

Die in der vorigen Reaktionsstufe erhaltenen 1,1 g wurden in Analogie zur Vorschrift in Beispiel 1q) zu 0,67 g Diol umgesetzt.

IR (Film): 3450, 2955, 2920, 2870, 1735, 1595, 1495, 1360, 1175, 978/cm.

11l) (5Z,13E)-(8R,9S,11R,12R,15S,16RS)-9-Azido-11,15-dihydroxy-16,20-dimethyl-5,13-prostadien-19-in-säuremethylester

650 mg des nach Beispiel 11k) hergestellten Tosylats wurden analog der Vorschrift des Beispiels 1r) mit Natriumazid umgesetzt. Man erhielt 360 mg der Titelverbindung als farbloses Öl.

IR (Film): 3400, 2960, 2930, 2870, 2110, 1735, 980/cm.

11m) (5Z,13E)-(8R,9S,11R,12R,15S,16RS)-9-Azido-11,15-dihydroxy-16,20-dimethyl-5,13-prostadien-19-in-säure

Die in der vorigen Reaktionsstufe erhaltenen 360 mg wurden in Analogie zum Beispiel 1s) verseift. Man erhielt 240 mg der Carbonsäure als Öl.

IR (Film): 3400 (breit), 2955, 2930, 2875, 2110, 1712, 978/cm.

**Patentansprüche für die Vertragsstaaten BE, CH, DE, FR, GB, IT, LI, LU, NL, SE**

1. Azaprostacycline der allgemeinen Formel I

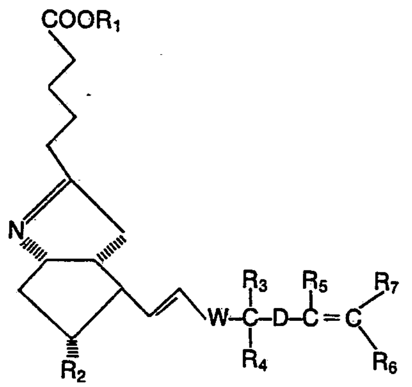

(I),

worin

$R_1$ Wasserstoff oder Alkyl mit 1–5 C-Atomen,

W eine CHOH-Gruppe, wobei die OH-Gruppe α- oder β-ständig sein kann,

$R_2$ eine Hydroxygruppe,

$R_3$ und $R_4$ Wasserstoff oder eine Alkylgruppe mit 1–5 C-Atomen,

D eine Alkylengruppe mit 1–2 C-Atomen,

$R_5$ Wasserstoff oder Alkyl mit 1–2 C-Atomen oder, wenn D eine Alkylengruppe mit 2 C-Atomen darstellt, zusammen mit $R_6$ eine Bindung,

$R_6$ und $R_7$ Wasserstoff oder Alkyl mit 1–2 C-Atomen,

$R_6(R_7)$ Halogen, wenn $R_7(R_6)$ Alkyl mit 1–2 C-Atomen darstellt oder

$R_5$ und $R_7$ Wasserstoff oder Alkyl mit 1–2 C-Atomen und

D und $R_6$ Glieder eines über $(CH_2)_{1-3}$ geschlossenen Ringes mit D als $-CH<$ und $R_6$ als $-CH_2-$ sind und,

falls $R_1$ die Bedeutung eines Wasserstoffatoms hat, deren Salze mit physiologisch verträglichen Basen.

2. Verfahren zur Herstellung von Azaprostacyclinen der allgemeinen Formel I, dadurch gekennzeichnet, dass man in an sich bekannter Weise eine Verbindung der allgemeinen Formel II

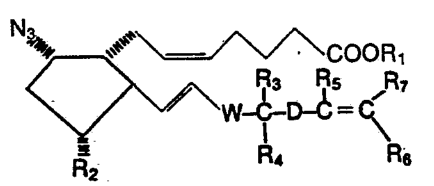

(II),

worin $R_1$, $R_2$, $R_3$, $R_4$, $R_5$, $R_6$, $R_7$, W und D die obengenannten Bedeutungen haben, in einem inerten Lösungsmittel einer thermischen Behandlung unterwirft und gegebenenfalls anschliessend in beliebiger Reihenfolge geschützte Hydroxygruppen freisetzt und/oder freie Hydroxygruppen verestert, veräthert und/oder eine freie Carboxylgruppe verestert und/oder eine veresterte Carboxylgruppe verseift oder eine Carboxylgruppe mit einer physiologisch verträglichen Base in ein Salz überführt.

3. Arzneimittel, bestehend aus einer oder mehreren Verbindungen gemäss Anspruch 1 und üblichen Hilfs- und Trägerstoffen.

4. (13E)-(11R,15S,16RS)-11,15-Dihydroxy-16,19-dimethyl-9α,6-nitrilo-13,18-prostadiensäure.

**Patentanspruch für den Vertragsstaat AT**

Verfahren zur Herstellung von Azaprostacyclinen der allgemeinen Formel I

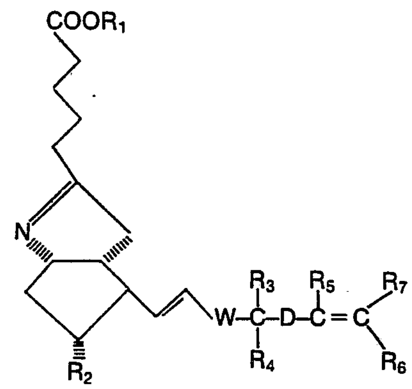

(I),

worin

$R_1$ Wasserstoff oder Alkyl mit 1–5 C-Atomen,

W eine CHOH-Gruppe, wobei die OH-Gruppe α- oder β-ständig sein kann,

$R_2$ eine Hydroxygruppe,

$R_3$ und $R_4$ Wasserstoff oder eine Alkylgruppe mit 1–5 C-Atomen,

D eine Alkylengruppe mit 1–2 C-Atomen,

$R_5$ Wasserstoff oder Alkyl mit 1–2 C-Atomen oder, wenn D eine Alkylengruppe mit 2 C-Atomen darstellt, zusammen mit $R_6$ eine Bindung,

$R_6$ und $R_7$ Wasserstoff oder Alkyl mit 1–2 C-Atomen,

$R_6(R_7)$ Halogen, wenn $R_7(R_6)$ Alkyl mit 1–2 C-Atomen darstellt oder

$R_5$ und $R_7$ Wasserstoff oder Alkyl mit 1–2 C-Atomen und

D und $R_6$ Glieder eines über $(CH_2)_{1-3}$ geschlossenen Ringes mit D als $-CH<$ und $R_6$ als $-CH_2-$ sind und,

falls $R_1$ die Bedeutung eines Wasserstoffatoms hat, deren Salze mit physiologisch verträglichen Basen

bedeuten, dadurch gekennzeichnet, dass man in an sich bekannter Weise eine Verbindung der allgemeinen Formel II

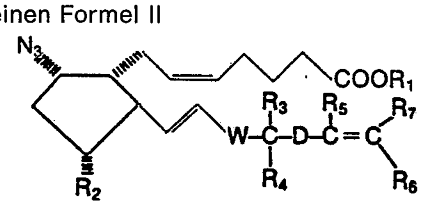

(II),

worin $R_1$, $R_2$, $R_3$, $R_4$, $R_5$, $R_6$, $R_7$, W und D die obengenannten Bedeutungen haben, in einem inerten Lösungsmittel einer thermischen Behandlung unterwirft und gegebenenfalls anschliessend in beliebiger Reihenfolge geschützte Hydroxygruppen freisetzt und/oder freie Hydroxygruppen verestert, veräthert und/oder eine freie Carboxylgruppe verestert und/oder eine veresterte Carboxylgruppe verseift oder eine Carboxylgruppe mit einer physiologisch verträglichen Base in ein Salz überführt.

**Patent Claims for the contracting states BE, CH, DE, FR, GB, IT, LI, LU, NL, SE**

1. Azaprostacyclins of the general formula I

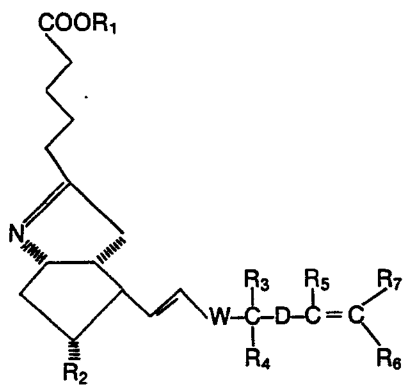

(I),

in which

$R_1$ represents hydrogen or alkyl having from 1 to 5 carbon atoms,

W represents a CHOH group in which the OH group may be in the α- or β-configuration,

$R_2$ represents a hydroxy group,

$R_3$ and $R_4$ represent hydrogen or an alkyl group having from 1 to 5 carbon atoms,

D represents an alkylene group having 1 or 2 carbon atoms,

$R_5$ represents hydrogen or alkyl having 1 or 2 carbon atoms or, when D represents an alkylene group having 2 carbon atoms, together with $R_6$ represents a bond,

$R_6$ and $R_7$ represent hydrogen or alkyl having 1 or 2 carbon atoms,

$R_6(R_7)$ represents halogen when $R_7(R_6)$ represents alkyl having 1 or 2 carbon atoms, or

$R_5$ and $R_7$ represent hydrogen or alkyl having 1 or 2 carbon atoms and

D and $R_6$ are members of a ring closed by $(CH_2)_{1-3}$, D representing $-CH<$ and $R_6$ representing $-CH_2-$,

and, if $R_1$ represents a hydrogen atom, salts thereof with physiologically tolerable bases.

2. Process for the manufacture of azaprostacyclins of the general formula I, characterised in that, in a manner known per se a compound of the general formula II

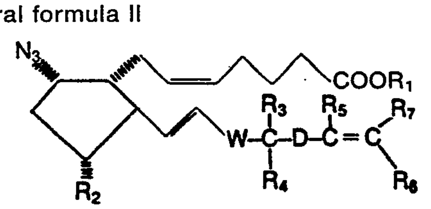

(II),

in which $R_1$, $R_2$, $R_3$, $R_4$, $R_5$, $R_6$, $R_7$, W and D have the meanings given above, is subjected to a thermal treatment in an inert solvent, and optionally then, in any sequence, protected hydroxy groups are freed and/or free hydroxy groups are esterified or etherified and/or a free carboxyl group is esterified and/or an esterified carboxyl group is hydrolysed or a carboxyl group is converted into a salt with a physiologically tolerable base.

3. Medicaments consisting of one or more compounds according to claim 1 and customary auxiliaries and carriers.

4. (13E)-(11R,15S,16RS)-11,15-dihydroxy-16,19-dimethyl-9α,6-nitrilo-13,18-prostadienoic acid.

**Claim for the contracting state AT**

Process for the manufacture of azaprostacyclins of the general formula I

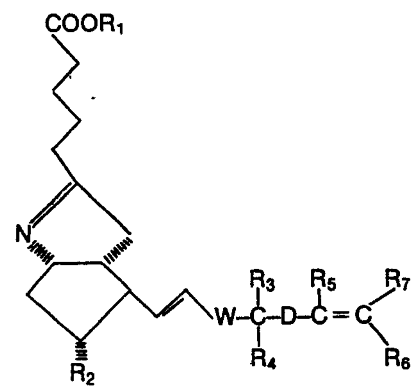

(I),

in which

$R_1$ represents hydrogen or alkyl having from 1 to 5 carbon atoms,

W represents a CHOH group in which the OH group may be in the α- or β-configuration,

$R_2$ represents a hydroxy group,

$R_3$ and $R_4$ represent hydrogen or an alkyl group having from 1 to 5 carbon atoms,

D represents an alkylene group having 1 or 2 carbon atoms,

$R_5$ represents hydrogen or alkyl having 1 or 2 carbon atoms or, when D represents an alkylene group having 2 carbon atoms, together with $R_6$ represents a bond,

$R_6$ and $R_7$ represent hydrogen or alkyl having 1 or 2 carbon atoms,

$R_6(R_7)$ represents halogen when $R_7(R_6)$ represents alkyl having 1 or 2 carbon atoms, or

$R_5$ and $R_7$ represent hydrogen or alkyl having 1 or 2 carbon atoms and

D and $R_6$ are members of a ring closed by $(CH_2)_{1-3}$, D representing $-CH<$ and $R_6$ representing $-CH_2-$,

and, if $R_1$ represents a hydrogen atom, salts thereof with physiologically tolerable bases, characterised in that, in a manner known per se a compound of the general formula II

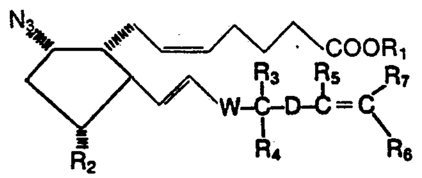

(II),

in which $R_1$, $R_2$, $R_3$, $R_4$, $R_5$, $R_6$, $R_7$, W and D have the meanings given above, is subjected to a thermal treatment in an inert solvent, and optionally then, in any sequence, protected hydroxy groups are freed and/or free hydroxy groups are esterified or etherified and/or a free carboxyl group is esterified and/or an esterified carboxyl group is hydrolysed or a carboxyl group is converted into a salt with a physiologically tolerable base.

**Revendications pour les Etats contractants BE, CH, DE, FR, GB, IT, LI, LU, NL, SE**

1. Azaprostacyclines répondant à la formule générale I:

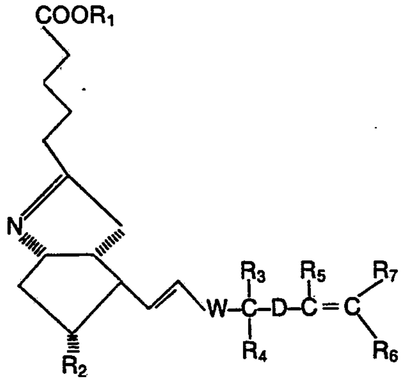

(I),

dans laquelle:

$R_1$ représente l'hydrogène ou un alkyle contenant de 1 à 5 atomes de carbone,

W représente un radical –CHOH dont le radical hydroxy peut avoir la configuration α ou la configuration β,

$R_2$ représente un radical hydroxy,

$R_3$ et $R_4$ représentent chacun l'hydrogène ou un alkyle en $C_1$–$C_5$,

D représente un alkylène à 1 ou 2 atomes de carbone,

$R_5$ représente l'hydrogène ou un alkyle à 1 ou 2 atomes de carbone, ou encore, lorsque D représente un alkylène à 2 atomes de carbone, $R_5$ et $R_6$ forment ensemble une liaison,

$R_6$ et $R_7$ représentent chacun l'hydrogène ou un alkyle à 1 ou 2 atomes de carbone,

$R_6$ (ou $R_7$) représente un halogène lorsque $R_7$ (ou $R_6$) représente un alkyle à 1 ou 2 atomes de carbone, ou

$R_5$ et $R_7$ représentent chacun l'hydrogène ou un alkyle à 1 ou 2 atomes de carbone et

D et $R_6$ sont des maillons d'un cycle fermé par $(CH_2)_{1-3}$, D sous la forme d'un maillon –CH< et $R_6$ sous la forme d'un maillon $-CH_2-$,

et, lorsque $R_1$ désigne un atome d'hydrogène, les sels qu'elles forment avec des bases acceptables du point de vue physiologique.

2. Procédé de préparation d'azaprostacyclines de formule générale I, procédé caractérisé en ce qu'on soumet à un traitement par la chaleur, de manière connue, un composé répondant à la formule générale II:

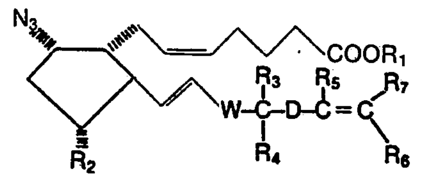

(II),

dans laquelle $R_1$, $R_2$, $R_3$, $R_4$, $R_5$, $R_6$, $R_7$, W et D ont les significations indiquées ci-dessus, dans un solvant inerte, puis, le cas échéant, en opérant dans l'ordre que l'on veut, on libère des radicaux hydroxy protégés et/ou on estérifie ou éthérifie des radicaux hydroxy libres et/ou on estérifie un radical carboxy libre et/ou on saponifie un radical carboxy estérifié, ou on transforme un radical carboxy en un sel avec une base acceptable du point de vue physiologique.

3. Médicament constitué d'un ou de plusieurs composés selon la revendication 1 et d'adjuvants et excipients usuels.

4. Acide dihydroxy-11,15 diméthyl-16,19 nitrilo-9α,6 prostadiène-13,18 oïque (13E)-(11R,15S,16RS).

**Revendication pour l'Etat contractant AT**

Procédé de préparation d'azaprostacyclines de formule générale I:

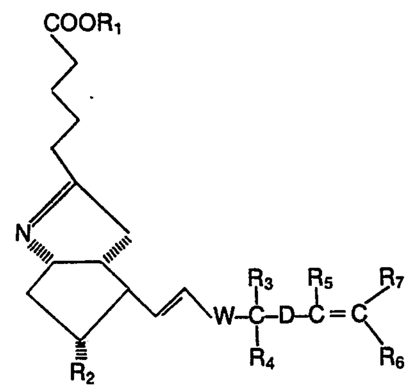

(I),

dans laquelle:

$R_1$ représente l'hydrogène ou un alkyle contenant de 1 à 5 atomes de carbone,

W représente un radical –CHOH dont le radical hydroxy peut avoir la configuration α ou la configuration β,

$R_2$ représente un radical hydroxy,

$R_3$ et $R_4$ représentent chacun l'hydrogène ou un alkyle en $C_1$–$C_5$,

D représente un alkylène à 1 ou 2 atomes de carbone,

$R_5$ représente l'hydrogène ou un alkyle à 1 ou 2 atomes de carbone, ou encore, lorsque D représente un alkylène à 2 atomes de carbone, $R_5$ et $R_6$ forment ensemble une liaison,

$R_6$ et $R_7$ représentent chacun l'hydrogène ou un alkyle à 1 ou 2 atomes de carbone,

$R_6$ (ou $R_7$) représente un halogène lorsque $R_7$ (ou $R_6$) représente un alkyle à 1 ou 2 atomes de carbone, ou

$R_5$ et $R_7$ représentent chacun l'hydrogène ou un alkyle à 1 ou 2 atomes de carbone et

D et $R_6$ sont des maillons d'un cycle fermé par $(CH_2)_{1-3}$, D sous la forme d'un maillon $-CH<$ et $R_6$ sous la forme d'un maillon $-CH_2-$,

et, lorsque $R_1$ désigne un atome d'hydrogène, les sels qu'elles forment avec des bases acceptables du point de vue physiologique, procédé caractérisé en ce qu'on soumet à un traitement par la chaleur, de manière connue, un composé répondant à la formule générale II:

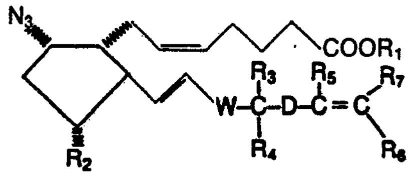
(II),

dans laquelle $R_1$, $R_2$, $R_3$, $R_4$, $R_5$, $R_6$, $R_7$, W et D ont les significations indiquées ci-dessus, dans un solvant inerte, puis, le cas échéant, en opérant dans l'ordre que l'on veut, on libère des radicaux hydroxy protégés et/ou on estérifie ou éthérifie des radicaux hydroxy libres et/ou on estérifie un radical carboxy libre et/ou on saponifie un radical carboxy estérifiè, ou on transforme un radical carboxy en un sel avec une base acceptable du point de vue physiologique.